# FASCICULE DE BREVET EUROPEEN

(11) **EP 1 339 679 B1**
(45) Date de publication et mention de la délivrance du brevet: **10.08.2005**
(21) Numéro de dépôt: 01997476.5
(22) Date de dépôt: 21.11.2001
(51) Int. Cl.: C07D 209/12, A61K 31/404, A61P 43/00

(54) **DERIVES DE 3-AROYLINDOLE ET LEUR UTILISATION EN TANT QU'AGONISTES DES RECEPTEURS CB2**
3-ARYLINDOLDERIVATE UND DEREN VERWENDUNG ALS CB2-REZEPTORAGONISTEN
3-ARYLINDOLE DERIVATIVES AND THEIR USE AS CB2 RECEPTOR AGONISTS

(30) Priorité: 22.11.2000 FR 0015158
(43) Date de publication de la demande: 03.09.2003
(73) Titulaire: Sanofi-Aventis, 75013 Paris (FR)
(72) Inventeur: BARTH, Francis, 34680 Saint-Georges d'Orques (FR); CONGY, Christian, F-34980 Saint-Gely-du-Fesc (FR); GUILLAUMONT, Carole, Fr-30700 Saint Quentin La Poterie (FR); RINALDI, Murielle, F-34680 Saint-Georges-d'Orques (FR); VASSE, Fabienne, Fr-34000 Montpellier (FR); VERNHET, Claude, Fr-34000 Montpellier (FR)
(74) Mandataire: Thouret-Lemaitre, Elisabeth
(86) Numéro de dépôt international: PCT/FR2001/003665
(87) Numéro de publication internationale: WO 2002/042269

(56) Documents cités:
- WO-A-01/28557
- WO-A-97/00860
- US-A- 5 532 237

## Description

La présente invention a pour objet de nouveaux composés dérivés de 3-aroylindole, agonistes des récepteurs aux cannabinoïdes CB₂, leur procédé de préparation et les compositions pharmaceutiques en contenant.

Le Δ⁹-THC est le principal constituant actif extrait de *Cannabis sativa* (Tuner, 1985 ; In Marijuana 1984, Ed. Harvey, DY, IRL Press, Oxford).

De nombreux articles ont décrit non seulement des effets psychotropes des cannabinoïdes mais aussi une influence de ces derniers sur la fonction immunitaire [HOLLISTER L.E. J. Psychoact. Drugs 24 (1992), 159-164]. La plupart des études *in vitro* ont montré des effets immunosuppresseurs des cannabinoïdes : l'inhibition des réponses prolifératives des lymphocytes T et des lymphocytes B induites par les mitogènes [Luo, Y.D. et al., Int. J. Immunopharmacol. (1992) 14, 49-56; Schwartz, H. et al., J. Neuroimmunol. (1994) 55, 107-115], l'inhibition de l'activité des cellules T cytotoxiques [Klein et al., J. Toxicol. Environ. Health (1991) 32, 465-477], l'inhibition de l'activité microbicide des macrophages et de la synthèse du TNFα [Arata, S. et al., Life Sci. (1991) 49, 473-479 ; Fisher-Stenger et al. J. Pharm. Exp. Ther. (1993) 267, 1558-1565], l'inhibition de l'activité cytolytique et de la production de TNFα des grands lymphocytes granulaires [Kusher et al. Cell. Immun. (1994) 154, 99-108]. Dans certaines études, des effets d'amplification ont été observés : augmentation de la bioactivité de l'interleukine-1 par les macrophages résidant de souris ou les lignées cellulaires macrophagiques différenciées, due à des niveaux accrus de TNFα [Zhu et al., J. Pharm. Exp. Ther. (1994) 270, 1334-1339 ; Shivers, S.C. et al. Life Sci. (1994) 54, 1281-1289].

Les effets des cannabinoïdes sont dus à une interaction avec des récepteurs spécifiques de haute affinité, couplés aux protéines G, présents au niveau central (Devane et al., Molecular Pharmacology (1988), 34, 605-613) et périphérique (Nye et al., J. Pharmacol. and Exp. Ther. (1985), 234, 784-791 ; Kaminski et al., Molecular Pharmacol. (1992), 42, 736-742 ; Munro et al., Nature (1993), 365, 61-65).

Les effets centraux des cannabinoïdes relèvent d'un premier type de récepteurs des cannabinoïdes (CB₁) qui est présent principalement dans le cerveau mais aussi à la périphérie. Par ailleurs, Munro et al. [Nature (1993) 365, 61-65] ont cloné un second type de récepteurs des cannabinoïdes, appelé CB₂, qui est présent à la périphérie et plus particulièrement sur les cellules d'origine immune. La présence de récepteurs aux cannabinoïdes CB₂ sur les cellules lymphoïdes peut expliquer l'immunomodulation exercée par les agonistes des récepteurs aux cannabinoïdes évoquée ci-dessus.

Certains dérivés indoliques ont été cités dans l'art antérieur comme présentant une affinité pour les récepteurs CB₂. Ainsi, le brevet US 5 532 237 décrit des composés de formule : 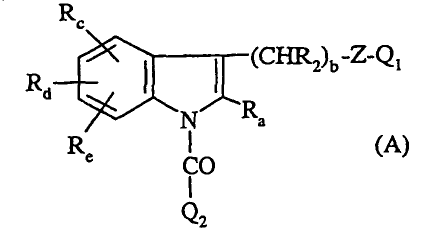 dans laquelle les substituants ont différentes valeurs ;
et la demande de brevet EP 833 818 décrit des composés de formule : 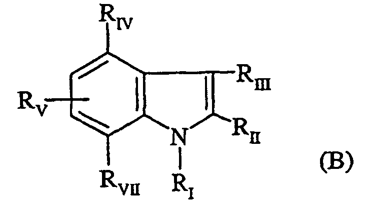 dans laquelle les substituants ont différentes valeurs.

La présente invention a pour objet des composés de formule : 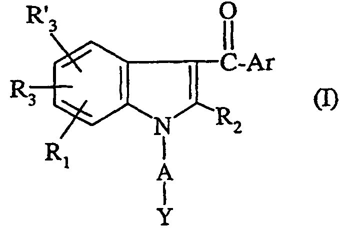 dans laquelle :
- Ar représente:
   a) un phényle mono, di ou trisubstitué par un ou plusieurs groupes choisis parmi :
      un atome d'halogène, un (C₁-C₄)alkyle, un trifluorométhyle, un amino, un nitro, un hydroxyle, un (C₁-C₄)alcoxy, un (C₁-C₄)alkylsulfanyle, un (C₁-C₄) alkylsulfonyle ;
   b) un naphtyle non substitué ou substitué une ou deux fois par un atome d'halogène, un (C₁-C₄)alkyle, un trifluorométhyle ;
- A représente un radical alkylène en C₂-C₆ ;
- Y représente un groupe choisi parmi SR₄, SOR₄, SO₂R₄, SO₂NR₅R₆, N(R₇)SO₂R₄, OR₄, NR₇SO₂NR₅R₆ ;
- R₁, R₃ et R'₃ représentent chacun indépendamment l'un de l'autre l'hydrogène, un hydroxyle, un atome d'halogène, un (C₁-C₄)alkyle, un trifluorométhyle, un (C₁-C₄)alcoxy ;
- R₂ représente l'hydrogène ou un (C₁-C₄)alkyle ;
- R₄ représente un (C₁-C₄)alkyle, un trifluorométhyle ;
- R₅ et R₆ représentent chacun indépendamment l'hydrogène ou un (C₁-C₄)alkyle ;
- R₇ représente l'hydrogène ou un (C₁-C₄)akyle ;
ainsi que leurs sels éventuels et leurs solvats.

Par halogène, on entend un atome de chlore, de brome, de fluor ou d'iode.

Par alkyle ou alkylidène, on entend un radical linéaire ou ramifié.

Lorsque les composés de formule (I) comprennent un atome de soufre ou un atome de carbone asymétrique, tous les isomères optiques ainsi que leur mélange en proportions quelconques sont objets de l'invention.

Les sels sont en général préparés avec des acides pharmaceutiquement acceptables mais les sels d'autres acides utiles pour la purification ou l'isolement des composés de formule (I) font également partie de l'invention. Les sels des composés de formule (I) pharmaceutiquement acceptables sont par exemple le chlorhydrate, le bromhydrate, le sulfate, l'hydrogénosulfate, le dihydrogénophosphate, le méthanesulfonate, le benzènesulfonate, le naphtalènesulfonate, le para-toluènesulfonate, le maléate, le fumarate, le succinate, le citrate, l'acétate, le gluconate, l'oxalate.

La présente invention a tout particulièrement pour objet des composés de formule: 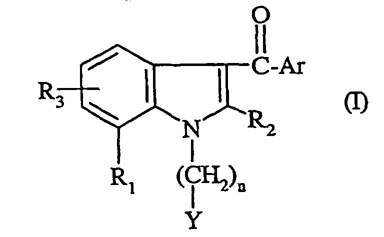 dans laquelle :
- Ar représente:
   a) un phényle mono, di ou trisubstitué par un ou plusieurs groupes choisis parmi :
      un atome d'halogène, un (C₁-C₄)alkyle, un trifluorométhyle, un amino, un nitro, un (C₁-C₄)alcoxy, un (C₁-C₄)alkylsulfanyle, un (C₁-C₄)alkylsulfonyle ;
   b) un naphtyle non substitué ou substitué une ou deux fois par un atome d'halogène, un (C₁-C₄)alkyle, un trifluorométhyle ;
- n représente 2, 3 ou 4 ;
- Y représente un groupe choisi parmi SR₄, SOR₄, SO₂R₄, SO₂NR₅R₆, N(R₇)SO₂R₄, OR₄ ;
- R₁ représente un atome d'halogène, un (C₁-C₄)alkyle, un trifluorométhyle, un (C₁-C₄)alcoxy ;
- R₂ représente l'hydrogène ou un (C₁-C₄)alkyle ;
- R₃ représente l'hydrogène, un (C₁-C₄)alkyle ou un halogène ;
- R₄ représente un (C₁-C₄)alkyle ;
- R₅ et R₆ représentent chacun indépendamment l'hydrogène ou un (C₁-C₄)alkyle ;
- R₇ représente l'hydrogène ou un (C₁-C₄)alkyle ;
ainsi que leurs sels éventuels et leurs solvats.

Selon la présente invention, on préfère les composés de formule (I) dans laquelle R₁ est en position 7 du noyau indole et représente un méthyle, un atome de chlore ou de brome ainsi que les composés de formule (I) dans laquelle R₂ représente un (C₁-C₄)alkyle, particulièrement un méthyle.

Les composés de formule (I) dans laquelle R₃ est l'hydrogène ou R₃ est en position 6 du noyau indole et représente un atome de chlore ou un méthyle sont préférés.

Les composés de formule (I) dans laquelle R'₃ est l'hydrogène sont préférés.

Les composés de formule (I) dans laquelle Ar représente un phényle mono ou disubstitué par un atome d'halogène, un méthyle, un trifluorométhyle, un méthoxy, un méthylsulfanyle, un méthylsulfonyle sont préférés.

Les composés de formule (I) dans laquelle Y représente SO₂R₄ ou NH SO₂R₄ sont également préférés ; particulièrement lorsque R₄ représente un méthyle ou un éthyle.

Ainsi, on préfère tout particulièrement les composés de formule (I) dans laquelle :
- Ar représente un phényle mono ou disubstitué par un atome d'halogène, un méthyle, un trifluorométhyle, un méthoxy, un méthylsulfanyle, un méthylsulfonyle ;
- A représente un groupe (CH₂)ₙ ;
- n représente 2, 3 ou 4 ;
- Y représente SO₂R₄ ou NHSO₂R₄ ;
- R₁ représente un méthyle, un atome de chlore ou de brome, en position 7 du noyau indole ;
- R₂ représente un méthyle ;
- R₃ est l'hydrogène ou R₃ représente soit un atome de chlore, soit un méthyle, en position 6 du noyau indole ;
- R'₃ est l'hydrogène ;
- R₄ représente un méthyle ou un éthyle ;
   ainsi que leurs sels éventuels et leurs solvats.

La présente invention a également pour objet les procédés de préparation des composés de formule (I), de leurs sels éventuels et de leurs solvats.

Un procédé selon l'invention, appelé procédé A, est caractérisé en ce que :
a) on traite un indole de formule : 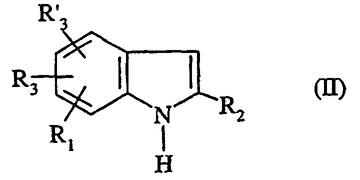 dans laquelle R₁, R₂, R₃ et R'₃ sont tels que définis pour un composé de formule (I), par un halogénure de méthylmagnésium et par un halogénure d'acide de formule ArCOHal (III), dans laquelle Ar est tel que défini pour le composé de formule (I) et Hal représente un atome d'halogène, de préférence le chlore ;
b) on traite le composé ainsi obtenu de formule : 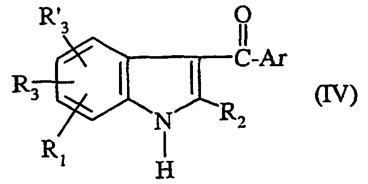 par un halogénure de formule Hal-A-Y (V) dans laquelle -A- et Y sont tels que définis pour un composé de formule (I) et Hal représente un atome d'halogène, de préférence le brome, en présence d'une base.

Le cas échéant, le composé de formule (I) ainsi obtenu, est transformé en l'un de ses sels ou solvats.

A l'étape a) du procédé ci-dessus, l'acylation est effectuée dans un solvant inerte tel que l'éther.

A l'étape b), on opère en présence d'une base telle que le carbonate de sodium ou le carbonate de potassium, un hydrure tel que l'hydrure de sodium ou un hydroxyde de métal alcalin tel que l'hydroxyde de potassium ; et dans un solvant tel que toluène, DMSO ou DMF, à une température comprise entre la température ambiante et la température d'ébullition du solvant. De façon particulière, lorsque la base utilisée est un hydroxyde de métal alcalin, on peut également effectuer l'étape b) en présence de tris[2-(2-méthoxyéthoxy)éthyl]amine (TDA-1), comme décrit dans Tetrahedron Lett., 1987, 28, 2963 ou d'un sel d'ammonium quaternaire, tel que l'hydrogénosulfate de tétrabutylammonium.

Il existe une variante de procédé A, appelée précédé A₁, caractérisé en ce que l'étape b) du procédé A est modifiée de la façon suivante :
b1) on traite le composé obtenu à l'étape a) de formule : 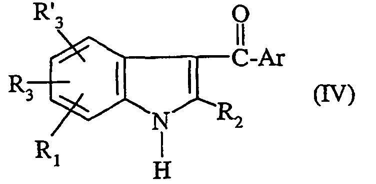 par un composé de formule Z-A-Cl (VI), dans laquelle Z représente soit un groupe hydroxyle soit un atome d'halogène, de préférence le brome ; et -A- est tel que défini pour (I) ;
b2) éventuellement, on traite le composé ainsi obtenu de formule : 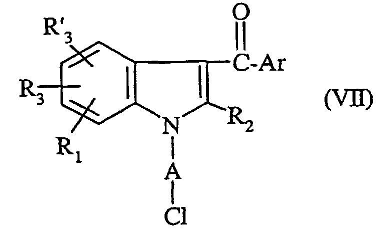 par l'iodure de sodium ;
b3) on traite le composé ainsi obtenu à l'étape b1 de formule (VII) ou à l'étape b2) de formule : 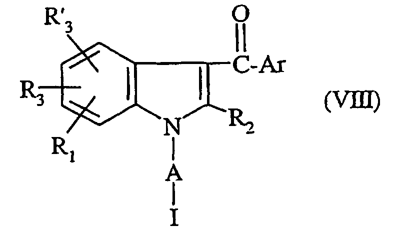 par un anion Y^{⊖}, Y étant tel que défini pour un composé de formule (I) pour former le composé de formule (I).

Lorsque Z représente un atome d'halogène, l'étape b1) est effectuée en présence d'une base, lorsque Z représente un groupe hydroxyle, l'étape b1) est effectuée en présence de triphénylphosphine et diéthylazodicarboxylate dans un solvant tel que le dichlorométhane.

A l'étape b2), lorsque celle-ci est réalisée, on utilise un solvant tel que l'acétonitrile, l'acétone ou un autre solvant cétonique.

Pour effectuer l'étape b3), on utilise un anion obtenu par réaction d'un composé de formule YH (IX) avec NaH dans un solvant tel que le DMF.

Le procédé A₁ est particulièrement préféré pour préparer des composés de formule (I) dans laquelle Y représente SR₄ ou NHSO₂R₄.

Selon une autre variante du procédé A, appelée procédé A₂, on peut préparer un composé de formule (I) dans laquelle Y représente un groupe SOR₄ ou un groupe SO₂R₄, à partir d'un composé de formule (I) dans laquelle Y représente un groupe SR₄. Ce procédé est caractérisé en ce que après l'étape b) du procédé A ou l'étape b2) ou b3) du procédé A₁, on effectue l'étape supplémentaire suivante :
c1) on traite le composé obtenu de formule : 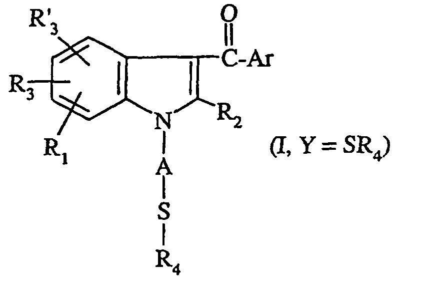 par un agent oxydant.

Comme agent oxydant, on peut utiliser l'eau oxygénée ou l'acide 3-chloroperbenzoïque ; selon le nombre d'équivalents d'oxydant utilisé et selon la température de la réaction, on obtient un sulfoxyde (I, Y = SOR₄) ou une sulfone (I, Y = SO₂R₄).

Selon une autre variante du procédé A, appelée procédé A₃, on peut préparer un composé de formule (I) dans laquelle Y représente un groupe N(R₇) SO₂R₄ dans laquelle R₇ est différent de H, à partir d'un composé de formule (I) dans laquelle Y représente un groupe NHSO₂R₄. Ce procédé est caractérisé en ce que, après l'étape b) du procédé A ou l'étape b2) ou b3) du procédé A₁, on effectue l'étape supplémentaire suivante :
c2) on traite le composé obtenu de formule : 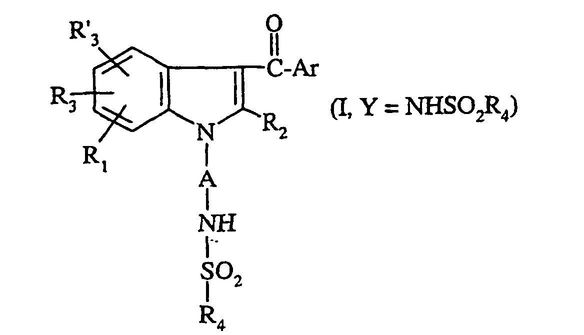 par un agent alkylant, en présence d'une base.

Comme agent alkylant on utilise, par exemple, un sulfate de dialkyle de formule SO₄(R₇)₂ ou un halogénure d'alkyle de formule R₇Hal, formules dans lesquelles R₇ est tel que défini pour les composés de formule (I) et Hal représente un atome d'halogène, de préférence l'iode, en présence d'une base, telle que l'hydrure de sodium par exemple.

Selon encore une autre variante du procédé A, appelé procédé A₄, on peut préparer un composé de formule (I) dans laquelle Y représente un groupe SO₂NR₅R₆ à partir d'un composé de formule (I) dans laquelle Y représente un groupe SO₂NHR₅. Ce procédé est caractérisé en ce que, après l'étape b) du procédé A ou l'étape b2) ou b3) du procédé A₁, on effectue l'étape supplémentaire suivante :
c3) on traite le composé obtenu de formule : 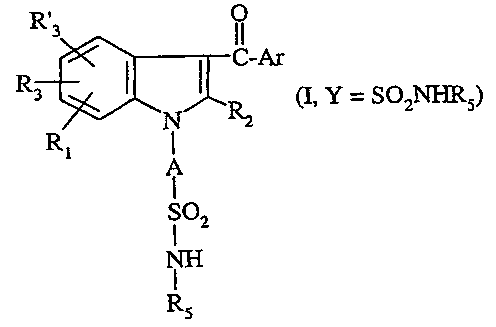 par un agent alkylant, en présence d'une base.

Comme agent alkylant on utilise, par exemple, un sulfate de dialkyle de formule SO₄(R₆)₂ ou un halogénure d'alkyle de formule R₆Hal, formules dans lesquelles R₆ est tel que défini pour les composés de formule (I) et Hal représente un atome d'halogène, de préférence l'iode, en présence d'une base telle que l'hydrure de sodium.

Lorsque l'on souhaite préparer un composé selon l'invention de formule (I) dans laquelle Y représente un groupe NR₇SO₂R₄ ou un groupe NR₇SO₂NR₅R₆, on peut utiliser une variante du procédé A, appelée procédé A5.

Ce procédé est caractérisé en ce que :
b4) on transforme le composé obtenu à l'étape b1) de formule : 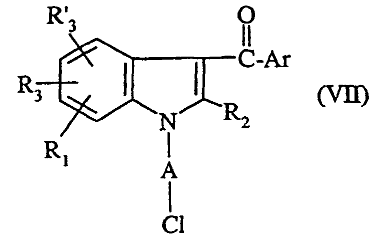 en un composé de formule : 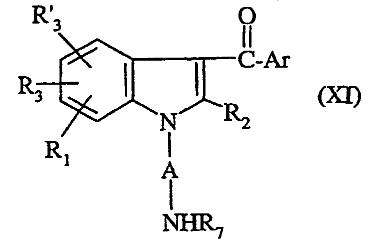 dans laquelle R₇ est tel que défini pour (I) ;
c4) on traite par un halogénure de formule HalSO₂R₄ ou respectivement HalSO₂NR₅R₆ dans laquelle R₄, R₅ et R₆ ont les significations données ci-dessus pour (I).

L'étape b4) peut être réalisée par différents procédés connus de l'homme de l'art, par exemple la réaction de Delépine (Synthesis, 1979, p. 161-179), la réaction de Gabriel (Angew. Chem. Int. Ed. Engl., 1998, 7, 919-930 ou la réaction d'Hebrard (Bull. Soc. Chim. Fr., 1970, 1938).

L'étape c4) peut être réalisée en présence d'une base telle que la triéthylamine.

Selon une méthode alternative du procédé A décrit ci-dessus et de ses variantes, on peut effectuer d'abord l'alkylation de l'azote indolique puis effectuer l'acylation du composé ainsi obtenu. Ce procédé alternatif, appelé procédé B, est caractérisé en ce que :
i) on traite un indole de formule : 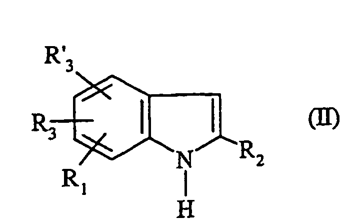 dans laquelle R₁, R₂, R₃ et R'₃ sont tels que définis pour le composé de formule (I) par un halogénure de formule Hal-A-Y (V) dans laquelle -A- et Y sont tels que définis pour un composé de formule (I) et Hal représente un atome d'halogène, de préférence le brome, en présence d'une base ;
ii) on traite le composé ainsi obtenu de formule : 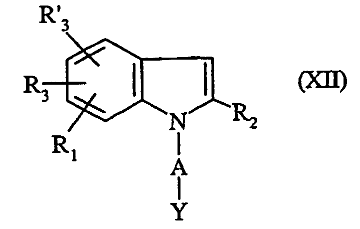 par un halogénure d'acide de formule ArCOHal (III) dans laquelle Ar est tel que défini pour le composé de formule (I) et Hal est un atome d'halogène, de préférence le chlore ou le brome.

Le cas échéant, le composé de formule (I) ainsi obtenu est transformé en l'un de ses sels ou solvats.

L'étape i) du procédé ci-dessus est réalisée dans les conditions décrites pour l'étape b) du procédé A. L'étape ii) est réalisée dans les conditions de Friedel et Crafts, en présence d'un acide de Lewis tel qu'AlCl₃ ou le dichlorure d'éthylaluminium dans un solvant inerte tel que le dichlorométhane ou le dichloroéthane, selon le procédé décrit dans J. Med. Chem., 1995, 38, 3094.

Il existe différentes variantes de l'étape i) du procédé B. Ces variantes correspondent à ce qui a été décrit pour le procédé A. Ces variantes constituent également un objet de la présente invention.

Ainsi la variante B₁ du procédé B est caractérisée en ce que :
i1) on traite un indole de formule : 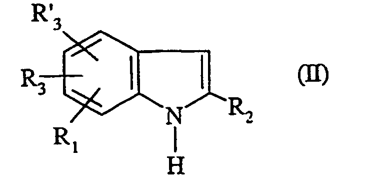 dans laquelle R₁, R₂, R₃ sont tels que définis pour le composé de formule (I), avec un composé de formule Z-A-Cl (VI) dans laquelle -A- est tel que défini pour le composé de formule (I) et Z représente un groupe hydroxyle ou un atome d'halogène, de préférence le brome ;
i2) éventuellement, on traite le composé ainsi obtenu de formule : 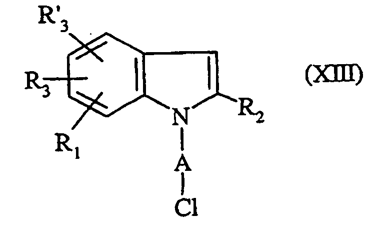 par l'iodure de sodium ;
i3) on traite le composé ainsi obtenu à l'étape i1) ou à l'étape i2) de formule : 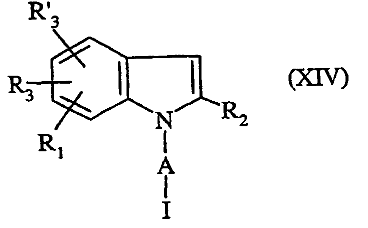 par un anion de formule Y⁻, Y étant tel que défini pour un composé de formule (I) ;
ii) on traite le composé ainsi obtenu de formule : 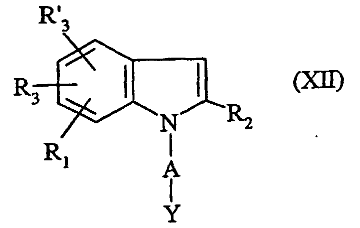 par un halogénure d'acide de formule ArCOHal (III) dans laquelle Ar est tel que défini pour le composé de formule (I) et Hal est un atome d'halogène, de préférence le chlore.

Le cas échéant, le composé de formule (I) ainsi obtenu est transformé en l'un de ses sels ou solvats.

De façon particulière, lorsque l'on souhaite préparer un composé de formule (I) dans laquelle A est (CH₂)₂, on peut utiliser des techniques connues de l'homme de l'art pour introduire la chaîne alkyle de longueur appropriée dans une des étapes, soit de la méthode A, soit de la méthode B.

Les indoles de formule (II) sont connus ou préparés par des méthodes connues telles que décrites dans J. Am. Chem. Soc., 1974, 96, 5495 et 1974, 96, 5512 ou dans Tetrahedron Lett., 1989, 30, 2129.

Les indoles de formule : 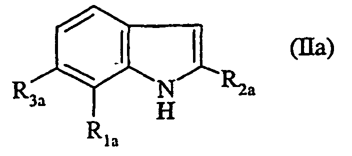 dans laquelle :
- R₁ₐ représente un atome de chlore ou de brome ou un méthyle ;
- R₂ₐ représente un (C₁-C₄)alkyle, de préférence le méthyle ;
- R₃ₐ représente l'hydrogène, un atome de chlore ou de brome ou un méthyle ;
   sont nouveaux et constituent un objet de la présente invention.

Les composés de formule ArCOCl (III) sont connus ou préparés par des méthodes connues.

Les composés de formule Hal(CH₂)ₙY (V) sont connus ou préparés par des méthodes connues. Par exemple, on peut préparer un ω-bromométhylsulfanylalkyle à partir d'un ω-hydroxyméthylsulfanylalkyle par action de PBr₃.

Les composés intermédiaires de formule : 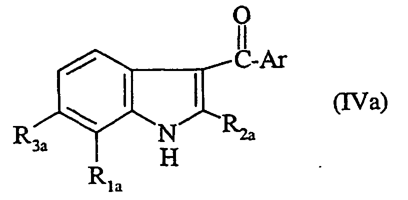 dans laquelle :
- R₁ₐ représente un atome de chlore ou de brome ou un méthyle;
- R₂ₐ représente un (C₁-C₄)alkyle, de préférence le méthyle ;
- R₃ₐ représente l'hydrogène, un atome de chlore ou de brome ou un méthyle ;
- Ar est tel que défini pour les composés de formule (I) ;
   sont nouveaux et constituent un objet ultérieur de l'invention.

Les composés intermédiaires de formule : 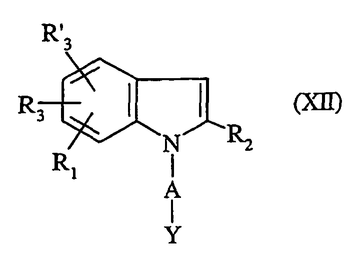 dans laquelle R₁, R₂, R₃, Y et A sont tels que définis pour (I) sont nouveaux et représentent un objet ultérieur de la présente invention.

Les composés selon l'invention ont montré une bonne affinité *in vitro* pour les récepteurs aux cannabinoïdes (CB₂) et une affinité *in vitro* nettement plus faible pour les récepteurs aux cannabinoïdes (CB₁) qu'il s'agisse de récepteurs humains ou de récepteurs de rongeur. Les essais de liaison par affinité (binding) ont été réalisés selon les conditions expérimentales décrites par Devane et al. (Molecular Pharmacology, 1988, 34, 605-613), avec des membranes issues de lignée cellulaires dans lesquelles les récepteurs CB₁ (Matsuda et al., Nature 1990, 346, 561-564) et CB₂ (Munro et al., Nature 1993, 365, 61-65) ont été exprimés. Pour les récepteurs humains, l'affinité *in vitro* aux cannabinoïdes CB₂ exprimé sous forme de Ki (constante d'inhibition) est de l'ordre du nM et le rapport entre l'affinité pour les récepteurs CB₁ et celle pour les récepteurs CB₂ est d'au moins 100.

D'autre part les composés selon l'invention se comportent *in vitro* comme des agonistes spécifiques des récepteurs humains aux cannabinoïdes CB₂ versus CB₁, ils diminuent la production d'AMPc dans les cellules stimulées par de la forskoline et ce en inhibant l'adénylate cyclase. Les essais ont été réalisés selon les conditions expérimentales décrites par Matsuda et al., Nature 1990, 346, 561-564.

Les composés selon l'invention possèdent également une affinité *in vivo* pour les récepteurs aux cannabinoïdes présents au niveau de la rate de souris lorsqu'ils sont administrés par voie orale. Les essais ont été réalisés selon les conditions expérimentales décrites par Rinaldi-Carmona et al., J. Pharmacol. Exp. Ther., 1998, 284, 644-650.

Ainsi les composés des exemples 7, 44, 46, 72, 89, 106, 120, 130, 132 pour lesquels le rapport entre l'affinité pour les récepteurs CB₁ et celle pour les récepteurs CB₂ est compris entre 400 et 4000 sont actifs par voie orale avec une DE₅₀ comprise entre 0,2 mg/kg et 20 mg/kg.

Les composés de la présente invention sont notamment des principes actifs de compositions pharmaceutiques dont la toxicité est compatible avec leur utilisation en tant que médicaments.

Selon un de ses aspects, la présente invention concerne l'utilisation d'un composé de formule (I) ou d'un de ses sels ou solvats, pharmaceutiquement acceptable pour la préparation de médicaments destinés à prévenir ou à traiter toute pathologie dans laquelle les récepteurs aux cannabinoïdes CB₂ sont impliqués.

On peut par exemple citer les maladies ou affections suivantes :
les désordres du système immunitaire, notamment les maladies autoimmunes : psoriasis, lupus érythémateux, maladies du tissu conjonctif ou connectivites, syndrome de Sjögrer's, spondylarthrite ankylosante, arthrite rhumatoïde, arthrite réactionnelle, spondylarthrite indifférenciée, maladie de Behcet's, anémies autoimmunes hémolytiques, sclérose en plaques, sclérose latérale amyotrophique, amyloses, le rejet de greffe, les maladies affectant la lignée plasmocytaire ; les maladies allergiques: hypersensibilité retardée ou immédiate, rhinite allergique, dermatite de contact, conjonctivite allergique ; les maladies infectieuses parasitaire, virale ou bactérienne : SIDA, méningites ; les maladies inflammatoires, notamment les maladies articulaires : arthrite, arthrite rhumatoïde, ostéoarthrite, spondylite, goutte, vascularite, maladie de Crohn, maladie du colon irritable syndrome de colon irritable (an anglais IBD : inflammatory bowel disease et IBS : irritable bowel syndrome) ; l'ostéoporose ; la douleur : les douleurs chroniques de type inflammatoire, les douleurs neuropathiques, les douleurs aigues périphériques ; les affections oculaires : hypertension oculaire, glaucome ; les affections pulmonaires : maladies des voies respiratoires, asthme, bronchite chronique, obstruction chronique des voies respiratoires (en anglais COPD : chronic obstructive pulmonary disease), emphysème; ; les maladies du système nerveux central et les maladies neurogénératives : syndrome de Tourette, maladie de Parkinson, maladie d'Alzheimer, démence sénile, chorée, chorée de Huntington, épilepsie, psychoses, dépression, lésions de la moëlle épinière ; la migraine, les vertiges, les vomissements, les nausées en particulier ceux consécutifs à une chimiothérapie ; les maladies cardiovasculaires en particulier hypertension, artériosclérose, crise cardiaque, ischémie cardiaque ; l'ischémie rénale ; les cancers : les tumeurs bégnines de la peau, papillomes et tumeurs cancéreuses, les tumeurs de la prostate, les tumeurs cérébrales (glioblastomes, médullo-epithéliomes, médulloblastomes, neuroblastomes, tumeurs d'origine embryonnaires, astrocytomes, astroblastomes, épendymomes, oligodendrogliomes, tumeur du plexus, neuro-epithéliomes, tumeur de l'épiphyse, épendymoblastomes, neuroectodermique, méningiomes malins, sarcomatoses, mélanomes malins, schwénnomes) ; les maladies gastro-intestinales ; l'obésité.

L'utilisation des composés selon l'invention pour la prévention et/ou le traitement des maladies ci-dessus mentionnées, ainsi que pour la préparation de médicaments destinés à traiter ces maladies fait partie intégrante de l'invention.

Les composés de formule (I) ci-dessus, ou l'un de leurs sels ou solvats pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Pour leur utilisation comme médicaments, les composés de formule (I) sont généralement administrés en unités de dosage. Lesdites unités de dosage sont de préférence formulées dans des compositions pharmaceutiques dans lesquelles le principe actif est mélangé avec un ou plusieurs excipients pharmaceutiques.

Ainsi, selon un autre de ses aspects, la présente invention concerne des compositions pharmaceutiques renfermant, en tant que principe actif, un composé de formule (I), ou l'un de ses sels ou solvats pharmaceutiquement acceptables.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Lorsque l'on prépare une composition solide sous forme de comprimés ou de gélules, on ajoute au principe actif, micronisé ou non, un mélange d'excipients pharmaceutiques qui peut être composé de diluants comme par exemple le lactose,le mannitol, la cellulose microcristalline, l'amidon, le phosphate dicalcique, de liants comme par exemple la polyvinylpyrrolidone, l'hydroxypropylméthylcellulose, des délitants comme la polyvinylpyrrolidone réticulée, la carboxyméthylcellulose réticulée, la croscarmellose de sodium, des agents d'écoulement comme la silice, le talc, des lubrifiants comme le stéarate de magnésium, l'acide stéarique, le tribéhénate de glycérol, le stéarylfumarate de sodium.

Des agents mouillants ou tensioactifs tels que le laurylsulfate de sodium, le polysorbate 80, le poloxamer 188 peuvent être ajoutés à la formulation.

Les comprimés peuvent être réalisés par différentes techniques, compression directe, granulation sèche, granulation humide, fusion à chaud (hot-melt).

Les comprimés peuvent être nus ou dragéifiés (par du saccharose par exemple) ou enrobés avec divers polymères ou autres matières appropriés.

Les comprimés peuvent avoir une libération flash, retardée ou prolongée en réalisant des matrices polymériques ou en utilisant des polymères spécifiques au niveau du pelliculage.

Les gélules peuvent être molles ou dures, pelliculées ou non de manière à avoir une activité flash, prolongée ou retardée (par exemple par une forme entérique).

Elles peuvent contenir non seulement une formulation solide formulée comme précédemment pour les comprimés mais aussi des liquides ou des semi-solides.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un tensioactif hydrophile tel que le polysorbate 80 ou le poloxamer 188. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres, des sprays.

Pour l'administration transdermique, on peut utiliser des patches sous forme multilaminée ou à réservoir dans lequel le principe actif peuvent être en solution alcoolique, des sprays.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane, des substituts de fréon ou tout autre gaz propulseur biologiquement compatible ; on peut également utiliser un système contenant le principe actif seul ou associé à un excipient, sous forme de poudre.

Le principe actif peut être également présenté sous forme de complexe avec une cyclodextrine, par exemple, α, β, γ-cyclodextrine, 2-hydroxypropyl-β-cyclodextrine.

Le principe actif peut être formulé également sous forme de microcapsules ou microsphères, éventuellement avec un ou plusieurs supports ou additifs.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,1 à 1000 mg de principe actif, de préférence de 0,5 à 250 mg devant être administrés une à quatre fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Les compositions de la présente invention peuvent contenir, à côté des composés de formule (I), ou de l'un de leurs sels, solvats et/ou hydrates pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention:

Les composés selon l'invention pourront également être utilisés pour la préparation de compositions à usage vétérinaire.

De plus, les composés selon l'invention, tel quel ou sous forme radiomarquée peuvent être utilisés comme outils pharmacologiques chez l'homme ou chez l'animal, pour la détection et la marquage des récepteurs aux cannabinoïdes CB₂.

Les PREPARATIONS et EXEMPLES suivants illustrent l'invention sans toute fois la limiter.

Dans les Préparations et dans les Exemples on utilise les abréviations suivantes :
éther : éther diéthylique
éther iso : éther diisopropylique
DMF : N,N-diméthylformamide
THF : tétrahydrofurane
DCM : dichlorométhane
AcOEt : acétate d'éthyle
TDA-1 : tris[2-(2-méthoxyéthoxy)éthyl]amine
Ether chlorhydrique : solution saturée d'acide chlorhydrique dans l'éther diéthylique
Triton B : N-benzyltriméthylammonium hydroxyde
F : point de fusion
TA : température ambiante
Eb : température d'ébullition

Les spectres de résonance magnétique du proton (RMN ¹H) sont enregistrés à 200 MHz dans du DMSO-d₆, en utilisant le pic du DMSO-d₆ comme référence. Les déplacements chimiques δ sont exprimés en parties par million (ppm). Les signaux observés sont exprimés ainsi : s: singulet ; se : singulet élargi ; d : doublet ; d.d : doublet dédoublé ; t : triplet ; td : triplet dédoublé ; q : quadruplet ; qt : quintuplet ; m : massif ; mt : multiplet ; sp : septuplet.

### Préparation des indoles de formule (II).

### Préparation 1.1

2-méthyl-7-chloro-1*H*-indole.

Sous atmosphère d'azote, on place 42,1 g de 2-chloronitrobenzène dans 850 ml de THF. On refroidit à -40°C puis on ajoute goutte à goutte, 1,6 1 de bromure d'isopropènylmagnésium 0,5M dans le THF. Après 1 heure à -40°C sous agitation, on hydrolyse par 400 ml d'une solution saturée de NH₄Cl. La phase aqueuse est extraite 2 fois à l'éther. On sèche puis évapore la phase organique et le résidu est chromatographié sur silice en éluant par du toluène. On obtient 20,3 g du composé attendu.

RMN : δ (ppm) : 2,4 : s : 3H ; 6,2 : s : 1H ; 6,9 : t : 1H ; 7,1 : d : 1H ; 7,4 : d : 1H ; 11,2 : se : 1H.

### Préparation 1.2

2-méthyl-7-isopropyl-1*H*-indole.

On prépare 1570 ml de solution 0,5 M de bromure d'isopropénylmagnésium dans le THF que l'on refroidit à -45°C. On coule lentement cette solution refroidie sur une solution de 43,3 g de 2-isopropylnitrobenzène dans 400 ml de THF placée sous azote. On refroidit le milieu réactionnel à -40°C puis on maintient sous agitation à cette température pendant 1 heure et demie. On verse le milieu réactionnel sur 1 litre de solution saturée de NH₄Cl. La phase aqueuse est extraite 2 fois à l'éther puis au DCM ; on lave par une solution saturée de NaCl. La phase organique est séchée puis évaporée et le résidu est chromatographié sur silice en éluant par un mélange AcOEt/cyclohexane (2/8 ; v/v). On obtient 23,7 g du composé attendu.

RMN : δ (ppm) : 1,1 : d : 6H ; 2,4 : s : 3H ; 3,3 : mt : 1H ; 6 : s : 1H ; 6,7 et 7,2 : 2mt : 3H ; 10,8 : se : 1H.

### Préparation 1.3

7-Bromo-2-éthyl-1*H*-indole.

On place 2,4 g de magnésium dans un ballon et on le recouvre par 10 ml de THF. On ajoute 1 g de 2-bromo-1-butène puis 10 ml de THF, puis à nouveau, 12,5 g de 2-bromo-1-butène dans 30 ml de THF. On observe un échauffement du milieu réactionnel, puis on chauffe à 60°C pendant 30 minutes après la fin de la réaction d'addition. On refroidit ensuite à -20°C, on ajoute 20 ml de THF puis on ajoute 6,7 g de 2-bromonitrobenzène à -20°C. On laisse revenir à TA. Le milieu réactionnel est versé sur 200 ml d'une solution de NaCl saturée. On extrait à l'éther puis on évapore et on reprend par DCM. La phase organique est lavée à l'eau puis par une solution saturée de NaCl. On sèche, évapore puis, le résidu est chromatographié sur silice en éluant par un mélange cyclohexane/AcOEt (9,5/0,5 ; v/v). On obtient 3,45 g du composé attendu.

RMN : δ (ppm) : 1,3 : t : 3H ; 2,8 : q : 2H ; 6,2 : s : 1H ; 6,8 : t : 1H ; 7,2 : d : 1H ; 7,4 : d : 1H ; 11 : se : 1H.

### Préparation 1.4

2-Méthyl-7-éthyl-1*H*-indole.

A 1,6 1 de bromure d'isopropènylmagnésium 0,5M dans le THF, on ajoute goutte à goutte, une solution de 36,5 g de 2-éthylnitrobenzène dans 250 ml de THF à -20°C. On maintient le milieu réactionnel à -20°C sous agitation pendant 2 heures puis on verse le milieu sur 800 ml de solution saturée de NaCl. On laisse décanter puis on extrait à l'éther. On sèche et évapore puis le résidu est chromatographié sur silice en éluant par un mélange AcOEt/cyclohexane (1/9 ; v/v). On obtient 23,75 g du composé attendu.

RMN : δ (ppm) : 1,3 : t : 3H ; 2,4 : s : 3H ; 2,8 : q : 2H ; 6,2 : s : 1H ; 6,8 : m : 2H ; 7,2 : d : 1H ; 10,8 : se : 1H.

### Préparation 1.5

7-Bromo-2-méthyl-1*H*-indole.

On place 27,0 g de 2-bromonitrobenzène dans 400 ml de THF. On place le milieu sous azote et on refroidit à -55°C puis on ajoute goutte à goutte, 800 ml de bromure d'isopropénylmagnésium 0,5 M dans le THF. On laisse sous agitation pendant 1 heure puis on verse le milieu dans une solution saturée de NH₄Cl. On extrait à l'éther, évapore puis reprend au DCM. On lave par une solution saturée de NaCl. On sèche et évapore puis le résidu est chromatographié sur silice en éluant par un mélange AcOEt/cyclohexane (1/9 ; v/v). On obtient 10,7 g du composé attendu.

RMN : δ (ppm) : 2,4 : s : 3H ; 6,2 : s : 1H ; 6,9 : t : 1H ; 7,2 : d : 1H ; 7,4 : d : 1H ; 11,2 : se : 1H.

### Préparation 1.6

6,7-dichloro-2-méthyl-1*H*-indole.

On introduit sous azote 1600 ml de bromure d'isopropénylmagnésium 0,5 M dans le THF, on refroidit à -20°C et ajoute 51,2 g de 2,3-dichloronitrobenzène dans 250 ml de THF anhydre puis on laisse 1 heure sous agitation à -20°C. On verse le milieu réactionnel à -20°C sur 1 litre de solution saturée de NH₄Cl, on dilue avec Et₂O puis on lave 2 fois la phase aqueuse avec Et₂O. On réunit les phases organiques que l'on concentre à sec. On extrait au DCM, lave 2 fois à l'eau puis par une solution saturée de NaCl. On sèche puis évapore et on chromatographie le résidu sur un mélange hexane/AcOEt (95/5 ; v/v). On obtient 24,27 g du composé attendu, F = 70-71°C

On a également préparé les dérivés d'indole décrits dans le tableau 1 ci-après :

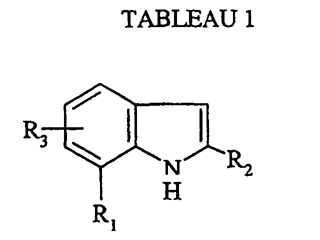

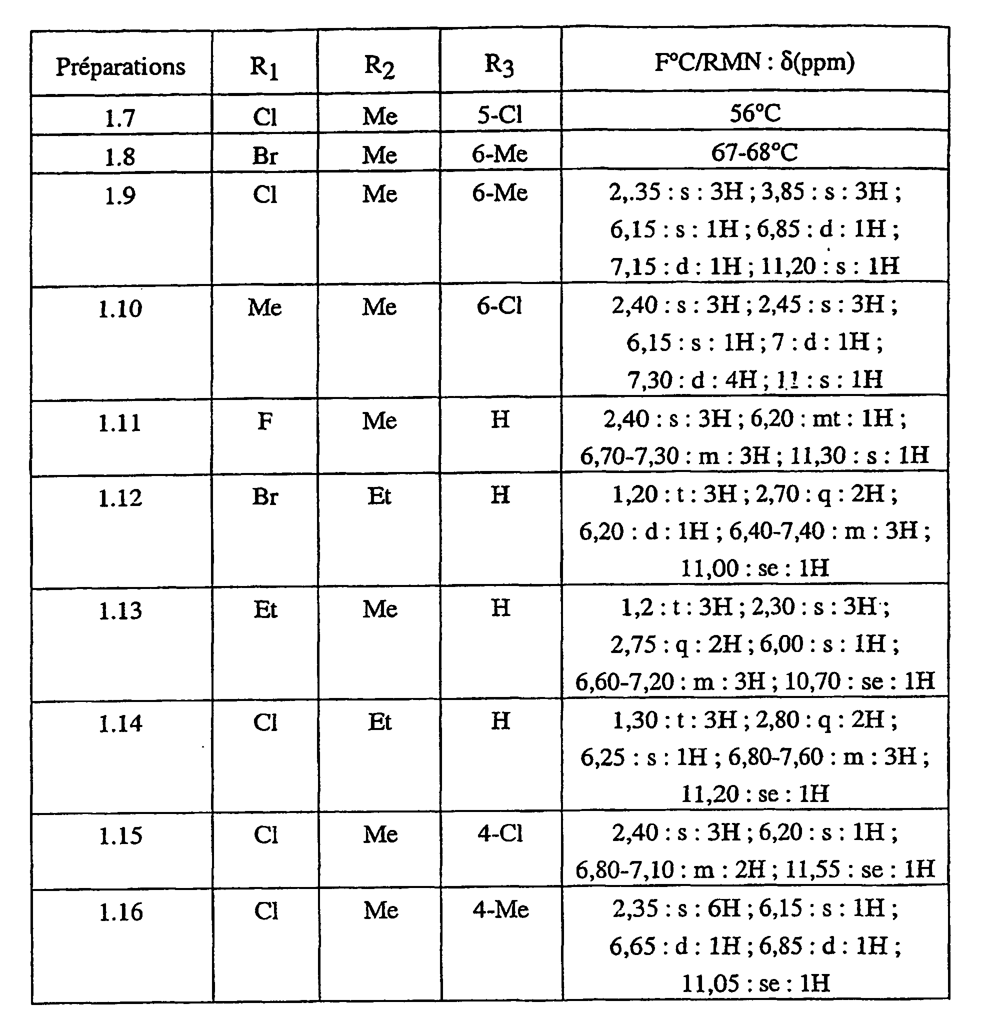

### EXEMPLE 1 (Procédé B)

(7-Chloro-2-méthyl-1-(3-(méthylsulfanyl)propyl)-1*H*-indol-3-yl)-(2,3-dichlorophényl)méthanone.
I : R₁ = Cl, R₂ = Me, R₃ = R'₃ = H, Ar = 2,3-dichlorophényle, Y = -S-Me, A = (CH₂)ₙ, n = 3.

### A) 1-Bromo-3-méthylsulfanylpropane.

Sous agitation, à 0°C, on mélange 24 g de PBr₃ à 20 g de 3-(méthylsulfanyl)-1-propanol. On laisse revenir à TA puis on chauffe le milieu à 100°C pendant 1 heure. On laisse refroidir à TA puis on refroidit dans un bain de glace. On reprend au benzène puis on extrait par du toluène. On lave par une solution à 1 % de Na₂CO₃, par de l'eau puis par une solution saturée de NaCl. On sèche sur MgSO₄, concentre puis distille pour obtenir 3,6 g du composé attendu.

### B) 7-Chloro-2-méthyl-1-(3-(méthylsulfanyl)propyl)-1H-indole.

On mélange sous agitation 1,5 g de 7-chloro-2-méthyl-1*H*-indole avec 1,1 g de potasse broyée et 0,2 g de TDA₁ On laisse 2 heures sous agitation à TA puis on ajoute goutte à goutte, 3,1 g de 1-bromo-3-méthylsulfanylpropane et on chauffe à reflux pendant 24 heures. On ajoute à nouveau de la potasse broyée (0,5 g), TDA-1 (0,1 g) et 1,6 g de 1-bromo-3-méthylsulfanylpropane. Après 9 heures de chauffage à reflux, on ajoute à nouveau 1,6 g de 1-bromo-3-méthylsulfanylpropane et on chauffe 24 heures à reflux. On laisse revenir à TA puis on extrait au toluène. La phase organique est lavée par une solution d'HCl à 10 %, de l'eau, puis une solution saturée de NaCl. On sèche, évapore, puis le résidu est chromatographié sur silice en éluant par un mélange cyclohexane/toluène (50/50 ; v/v). On obtient 1,87 g du composé attendu.
RMN : δ (ppm) : 1,9 : mt : 2H ; 2,1 : s : 3H ; 2,4 : s : 3H ; 2,5 : mt : 2H ; 4,5 : t : 2H ; 6,3 : s : 1H ; 6,9 : t : 1H ; 7,1 : d : 1H ; 7,4 : d : 1H.

### C) (7-Chloro-2-méthyl-1-(3-(méthylsulfanyl)propyl)-1H-indol-3-yl)-(2,3-dichlorophényl)méthanone.

Sous atmosphère d'azote, on mélange en agitant 1,87 g du composé de l'étape précédente dans 50 ml de DCM et 2,06 g de chlorure de 2,3-dichlorobenzoyle. On abaisse la température à -5°C puis on ajoute goutte à goutte, 9 ml de dichloroéthylaluminium 1,8M dans le toluène. On laisse revenir à TA et on maintient sous agitation pendant 24 heures. Le milieu est extrait au DCM, la phase organique est lavée par une solution saturée de NH₄Cl, de l'eau puis une solution saturée de NaCl. On sèche et évapore puis le résidu est chromatographié sur silice en éluant par un mélange cyclohexane/toluène (50/50 ; v/v). Le produit obtenu est cristallisé dans un mélange éthanol/heptane. On obtient 900 mg du composé attendu, F = 87°C.
RMN : δ (ppm) : 2 : qt : 2H ; 2,1 : s : 3H ; 2,4 : s : 3H ; 2,6 : t : 2H ; 4,6 : mt : 2H ; 7 à 7,9 : m : 6H.

### EXEMPLE 2 (Procédé A₂)

(7-Chloro-2-méthyl-1-(3-(méthylsulfonyl)propyl)-1*H*-indol-3-yl)-2,3-dichlorophényl)méthanone.
I : R₁ = CI, R₂ = Me, R₃ = R'₃ = H, Ar = 2,3-dichlorophényle, Y = SO₂Me, A = (CH₂)ₙ, n = 3.

On place 0,73 g du composé de l'Exemple 1 dans 5 ml de DCM. On abaisse la température à 0°C puis on ajoute goutte à goutte, 1,03 g d'acide 3-chloroperbenzoïque dilué dans 10 ml de DCM et on laisse sous agitation pendant 24 heures à TA. Le milieu est extrait au DCM puis on lave par une solution de Na₂CO₃ à 5 %, par de l'eau puis par une solution saturée de NaCl. On sèche et évapore ; le produit obtenu est cristallisé dans un mélange éther/hexane. On obtient 530 mg du composé attendu, F = 127°C.
RMN : δ (ppm) : 2,2 : mt : 2H ; 2,4 : s : 3H ; 3,0 : s : 3H ; 3,3 : mt : 3H ; 4,7 : t : 2H ; 7,1 : t : 1H ; 7,2 à 7,6 : m : 4H ; 7,8 : dd : 1H.

### EXEMPLE 3 (Procédé B₁)

N-(3-(7-Chloro-3-(2-fluoro-3-trifluorométhylphényl)-2-méthyl-1*H*-indol-1-yl)propyl)méthanesulfonamide.
I : R₁ = Cl, R₂ = Me, R₃ = R'₃ = H, Ar = 2-fluoro-3-trifluorométhylphényle, Y = NHSO₂Me, A = (CH₂)ₙ, n = 3.

### A) 7-chloro-1-chloropropyl-2-méthyl-1H-indole.

Sous azote, on place 40 g de 7-chloro-2-méthyl-1*H*-indole dans 60 ml de toluène avec 2,8 g de KOH. Après 30 minutes sous agitation à TA, on ajoute 7,7 g de 3-chloro-1-bromopropane puis on chauffe à reflux pendant 3 heures. Le milieu est extrait à l'éther. La phase organique est lavée à l'eau, par une solution d'HCl à 10 %, à l'eau, par une solution saturée de NaCl. On sèche, on évapore et on obtient 6,19 g du composé attendu.

### B) N-(3-(7-Chloro-2-méthyl-1H-indol-1-yl)propyl)méthanesulfonamide

Sous azote, on prépare un mélange contenant 2,2 g de NaH à 60 % dans l'huile et 170 ml de DMF que l'on refroidit à 0°C. On ajoute 4,0 g de NH₂SO₂CH₃ puis on laisse revenir à TA et on ajoute 5,0 g du composé de l'étape précédente. On chauffe à 130°C pendant 6 heures. Le milieu est extrait au DCM, la phase organique est lavée à l'eau puis par une solution de NaCl saturée. On sèche, on évapore, puis le résidu est chromatographié sur silice en éluant par un mélange cyclohexane/AcOEt (30/70 ; v/v). On obtient 1,92 g du composé attendu.

### C) N-(3-(7-Chloro-3-(2-fluoro-3-trifluorométhylphényl)-2-méthyl-1H-indol-1-yl)propyl)méthanesulfonamide.

Sous azote, on mélange 0,80 g du composé de l'étape précédente, 0,90 g de chlorure de 2-fluoro-3-trifluorométhylbenzoyle dans 60 ml de DCM. On abaisse la température à 0°C puis on ajoute 34 ml de dichloroéthylaluminium, 1,8 M dans le toluène. On laisse revenir à TA puis on agite le milieu pendant 15 heures. On extrait au DCM. La phase organique est lavée à l'eau, par une solution saturée de NaCl. On sèche, on évapore. Le produit est cristallisé dans un mélange DCM-éther. On obtient 550 mg du composé attendu, F = 168°C.
RMN : δ (ppm) : 2 : mt : 2H ; 2,4 : s : 3H ; 3,0 : s : 3H ; 3,2 : mt : 2H ; 4,7 : mt : 2H ; 7 à 7,5 : m : 3H ; 7,7 : t : 1H ; 8 : t : 1H ; 8,2 : t : 1H.

### EXEMPLE 4 (Procédé A)

(7-Isopropyl-2-méthyl-1-((2-éthylsulfanyl)éthyl)-1*H*-indol-3-yl)-(2,3-dichlorophényl)méthanone.
I : R₁ = iPr, R₂ = Me, R₃ = R'₃ = H, Ar = 2,3-dichlorophényle, Y = -S-Et, A = (CH₂)ₙ, n = 2

### A) (7-Isopropyl-2-méthyl-1H-indol-3-yl)-(2,3-dichlorophényl)méthanone.

On refroidit à -5°C 120 ml de THF anhydre et on ajoute 36 ml de bromure de méthylmagnésium 3M dans l'éther. On refroidit à -30°C puis on ajoute goutte à goutte, 15 g de 7-isopropyl-2-méthyl-1*H*-indole. On laisse 1 heure sous agitation entre -20°C et -30°C puis on ajoute goutte à goutte, 30,9 g de chlorure de 2,3-dichlorobenzoyle dissous dans 120 ml de THF. On laisse revenir à TA puis on verse le milieu sur 300 ml d'une solution saturée de NH₄Cl. On décante, évapore puis reprend au DCM. On lave par une solution saturée de NaCl puis on sèche et évapore. Le résidu est chromatographié sur silice en éluant par un mélange AcOEt/cyclohexane (3/7 ; v/v).

### B) (7-Isopropyl-2-méthyl-1-((2-éthylsulfanyl)éthyl)-1H-indol-3-yl)-(2,3-dichlorophényl)méthanone.

On mélange 0,7 g de soude pilée, 1,5 g du composé de l'étape précédente, 0,15 g d'hydrogénosulfate de tétrabutylammonium et 2,2 g de 1-chloro-1-éthylsulfanyléthane. On ajoute 60 ml de toluène puis 0,2 g d'eau et on chauffe à reflux pendant 3 jours. On refroidit le milieu puis on le verse sur de l'eau (200 ml). On extrait par 100 ml d'éther, lave à l'eau puis par une solution saturée de NaCl. On sèche et évapore puis le résidu est chromatographié sur silice en éluant par un mélange AcOEt/cyclohexane (15/85 ; v/v). On obtient 0,85 g du composé attendu.
RMN : δ (ppm) : 1,1 : t : 3H ; 1,25 : d : 6H ; 2,3 à 2,6 : m : 5H ; 2,8 : t : 2H ; 3,45 : sp : 1H ; 4,4 : t : 2H ; 6,9 à 7,8 : m : 6H.

### EXEMPLE 5 (Procédé A)

(7-Bromo-2-méthyl-1-(3-(méthylsulfanyl)propyl)-1*H*-indol-3-yl)-(2,3-dichlorophénylméthanone).
I : R₁ = Br, R₂ = Me, R₃ = R'₃ = H, Ar = 2,3-dichlorophényle, Y = S-CH₃,
A = (CH₂)ₙ, n = 3.

### A) (7-Bromo-2-méthyl-1H-indol-3-yl)-(2,3-dichlorophénylméthanone).

On place 10,7 g de 7-bromo-2-méthyl-1*H*-indole dans 100 ml de THF et on refroidit à -10°C. A cette température, on ajoute 22 ml de bromure de méthylmagnésium 3M dans l'éther. On laisse revenir à TA puis on refroidit à -5°C et on ajoute goutte à goutte, 13,5 g de chlorure de 2,3-dichlorobenzoyle dissous dans 80 ml de THF. On laisse revenir à TA puis on verse le milieu sur une solution saturée de NH₄Cl. On extrait à l'éther, puis on lave la phase organique par une solution de NaOH à 10 %, de l'eau, une solution saturée de NaCl. On sèche et évapore, puis le résidu est chromatographié sur silice en éluant par un mélange AcOEt/cyclohexane (10/90 ; v/v). Le produit obtenu cristallise dans l'éther, on obtient 5 g du composé attendu.

### B) (7-Bromo-2-méthyl-1-(3-chloropropyl)-1H-indol-3-yl)-(2,3-dichlorophényle) méthanone.

A 1 g de potasse pilée, on ajoute 3 g du composé de l'étape précédente, 0,3 g de TDA-1 et 100 ml de toluène puis on chauffe à reflux pendant 30 minutes et on ajoute 5 g de 1-bromo-3-chloropropane. On laisse refroidir le milieu à TA puis on le verse sur 100 ml d'une solution d'HCl à 10 %. Le milieu est extrait au toluène puis la phase organique est lavée à l'eau puis par une solution saturée de NaCl. On sèche et évapore puis on chromatographie le résidu sur silice en éluant au DCM. On obtient 3,25 g du composé attendu, F = 139°C.

### C) (7-Bromo-2-méthyl-1-(3-(méthylsulfanyl)propyl)-1H-indol-3-yl)-(2,3-dichlorophénylméthanone).

A TA, on mélange 3 g du composé de l'étape précédente et 0,62 g de MeSNa dans 40 ml d'éthanol. On chauffe à reflux pendant 2 heures et demie puis on laisse refroidir. On verse le milieu sur une solution de soude à 10 %. Le milieu est extrait à l'éther puis la phase organique est lavée par une solution saturée de NaCl. On obtient 2 g du composé attendu, F = 119°C.
RMN : δ (ppm) : 2 : mt : 2H ; 2,1 : s : 3H ; 2,4 : s : 3H ; 2,6 : t : 2H ; 4,6 : t : 2H ; 7 : t : 1H ; 7,4 à 7,6 : m : 4H ; 7,8 : dd: 1H.

### EXEMPLE 6 (Procédé A₂)

7-Bromo-2-méthyl-1-(3-(méthylsulfinyl)propyl)-1*H*-indol-3-yl)-(2,3-dichlorophényl)méthanone.
I : R₁ = Br, R₂ = Me, R₃ = R'₃ = H, Ar = 2,3-dichlorophényle, Y = -SOMe, A = (CH₂)ₙ, n = 3.

On place 2,5 g du composé de l'Exemple précédent dans 50 ml d'acide acétique et on refroidit à 10°C. On ajoute 0,8 ml d'H₂O₂ sous agitation puis on laisse revenir à TA et on maintient l'agitation pendant 1 heure et demie. On évapore puis on extrait le milieu par AcOEt. La phase organique est lavée par une solution de NaOH à 10 %, de l'eau, une solution saturée de NaCl. On évapore, le produit obtenu est recristallisé dans un mélange AcOEt/MeOH (9/1 ; v/v). On obtient 1,1 g du composé attendu, F = 137°C.
RMN : δ (ppm) : 2,1 : qt : 2H ; 2,4 et 2,6 : 2s : 6H ; 2,8 à 3,2 : mt : 2H ; 4,8 : t : 2H ; 7,1 : t : 1H ; 7,5 : m : 4H ; 7,9 : d : 1H.

### EXEMPLE 7 (Procédé A₂)

(7-Bromo-2-méthyl-1-(3-(méthylsulfonyl)propyl)-1*H*-indol-3-yl)-(2,3-dichlorophényl)méthanone.
I : R₁ = Cl, R₂ = Me, R₃ = R'₃ = H, Ar = 2,3-dichlorophényl, Y = SO₂Me, A = (CH₂)ₙ, n = 3.

On place 1,82 g d'acide 3-chloroperbenzoïque dans 40 ml de DCM et on refroidit à 0°C puis on ajoute goutte à goutte, 1,5 g du composé de l'Exemple 5 dissous dans 30 ml de DCM à 0°C. On laisse revenir à TA puis on maintient l'agitation pendant 2 heures. Après 48 heures au repos, on filtre le précipité formé (acide en excès) puis on verse le filtrat dans une solution de NaOH à 30 %. Le milieu est extrait 2 fois par Et₂O. La phase organique est lavée à l'eau puis par une solution saturée de NaCl. On sèche et évapore puis le résidu est chromatographié sur silice en éluant par AcOEt. On obtient
0,92 g du composé attendu, F = 90°C.
RMN : δ (ppm) : 2,2 : mt : 2H ; 2,4 : s : 3H ; 3 : s : 3H ; 3,3: t : 2H ; 4,7 : t : 2H ; 6,9 à 8 : m : 6H.

### EXEMPLE 8 (Procédé A₁)

N-(3-(7-Bromo-3-(2,3-dichlorobenzoyl)-2-éthyl-1*H*-indol-1-yl)propyl) méthanesulfonamide.
I : R₁ = Br, R₂ = Et, R₃ = R'₃ = H, Ar = 2,3-dichlorophényle, Y = NHSO₂Me, A = (CH₂)ₙ, n = 3.

### A) (7-Bromo-2-éthyl-1H-indol-3-yl)-(2,3-dichlorophényl)méthanone.

Sous azote, on place 3,45 g de 7-bromo-2-éthyl-1*H*-indole dans 30 ml d'éther et on refroidit à +3°C ; on ajoute 5,1 ml d'iodure de méthylmagnésium dans 20 ml d'éther puis 5,9 g de chlorure de dichlorobenzoyle dans 30 ml d'éther. On ajoute 10 ml de THF, laisse revenir à TA et place sous agitation pendant 3 heures. On verse le milieu sur une solution saturée de NH₄Cl puis on extrait à l'éther. La phase organique est lavée par une solution de HCl à 10 %, une solution de NaOH à 10 %, de l'eau, une solution de NaCl. On sèche, on évapore, puis on chromatographie le résidu sur silice en éluant par un mélange AcOEt/cyclohexane (3/7 ; v/v). On obtient 2,18 g du composé attendu.
RMN : δ (ppm) : 1 : t : 3H ; 2,8 : q : 2H ; 6,9 : t : 1H ; 7,1 : d : 1H ; 7,2 à 7,6 : m : 3H ; 7,8 : dd : 1H ; 12,2 : se : 1H.

### B) (7-Bromo-1-(3-chloropropyl)-2-éthyl-1H-indol-3-yl)-(2,3-dichlorophényl) méthanone.

On place 1,85 g du composé de l'étape précédente dans 100 ml de toluène avec 0,3 g de TDA-1, on introduit 1 g de potasse pilée puis on chauffe à reflux sous azote et l'on ajoute 3,2 g de 3-bromo-1-chloropropane dilué dans 15 ml de toluène. On chauffe une nuit à reflux puis on jette le milieu réactionnel sur de l'eau. On extrait au toluène, lave à l'eau puis par une solution saturée de NaCl. On sèche, évapore, puis on chromatographie le résidu sur silice en éluant par un mélange AcOEt/cyclohexane (3/7 ; v/v). On obtient 2 g du composé attendu.

### C) (7-Bromo-1-(3-iodopropyl)-2-éthyl-1H-indol-3-yl)-(2,3-dichlorophényl) méthanone.

On mélange 2,0 g du composé de l'étape précédente et 4,4 g de NaI dans 120 ml de CH₃CN puis on chauffe à 80°C pendant 5 jours. Il se forme un précipité de NaCl. On verse le milieu réactionnel sur de l'eau puis on extrait au toluène et lave par une solution saturée de NaCl. On obtient 2,4 g du composé attendu.

### D) N-(3-(7-Bromo-3-(2,3-dichlorobenzoyl)-2-éthyl-1H-indol-1-yl)propyl) méthanesulfonamide.

On ajoute 0,74 g de NaH à 60 % dans l'huile à 80 ml de DMF et l'on refroidit à +5°C. On ajoute 2 g de MeSO₂NH₂ dans 50 ml de DMF et on agite pendant 10 minutes. On ajoute alors 2,4 g du composé de l'étape précédente dans 40 ml de DMF et on laisse 3 heures sous agitation à +5°C. Le milieu réactionnel est jeté sur de l'eau puis on extrait au DCM. On lave à l'eau puis par une solution de NaCl concentrée. On sèche et évapore puis le résidu est chromatographié sur silice en éluant par AcOEt/cyclohexane (1/1). On obtient 1,1 g du composé attendu.
RMN : δ (ppm) : 1,05 : t : 3H ; 1,9 : mt : 2H ; 2,65 à 3,2 : m : 7H ; 4,5 : mt : 2H ; 6,7 à 7,8 : m : 6H.

### EXEMPLE 9 (Procédé A₃)

N-(3-(7-Bromo-3-(2,3-dichlorobenzoyl)-2-éthyl-1*H*-indol-1-yl)propyl)-N-méthylméthanesulfonamide.
I : R₁ = Br, R₂ = Et, R₃ = R'₃ = H, Ar = 2,3-dichlorophényl, Y = NMeSO₂Me, A = (CH₂)ₙ, n = 3.

On place 0,32 g de NaH dans 20 ml de DMF anhydre et on ajoute goutte à goutte, 0,86 g du composé de l'Exemple précédent dissous dans 20 ml de DMF. On observe un dégagement gazeux. On ajoute goutte à goutte, 1,15 g de MeI dans 20 ml de DMF et on laisse sous agitation pendant 2 heures. On verse le milieu réactionnel sur de l'eau puis on décante. On extrait au DCM puis on lave la phase organique par une solution saturée de NaCl. On sèche et évapore puis le résidu est chromatographié sur silice en éluant par un mélange AcOEt/cyclohexane (2/1 ; v/v). On obtient 0,5 g du composé attendu, F = 111°C.
RMN : δ (ppm) : 1,1 : t : 3H ; 1,95 : mt : 2H ; 2,6 à 3 : m : 8H ; 3,2 : t : 2H ; 4,5 : mt : 2H ; 6,8 à 7,9 : m : 6H.

### EXEMPLE 10 (Procédé A)

3-(3-(2,3-dichlorobenzoyl)-7-éthyl-2-méthyl-1*H*-indol-1-yl)-N,N-diméthyl-1-propanesulfonamide.
I : R₁ = Et, R₂ = Me, R₃ = R'₃ = H, Ar = 2,3-dichlorophényle, Y = -SO₂NMe₂, A = (CH₂)ₙ, n = 3.

### A) 3-Chloro-N,N-diméthyl-1-propanesulfonamide.

Le produit est préparé selon J. Am. Chem. Soc., 1951, 73, 3100.

On place 10 g de chlorhydrate de diméthylamine dans 60 ml d'eau, on met sous atmosphère d'azote et on refroidit entre 0°C et -5°C puis on ajoute goutte à goutte, 18 ml de chlorure de 3-chloro-1-propanesulfonyle. En maintenant à -5°C, on ajoute 10,6 g de soude dans 40 ml d'eau puis on laisse revenir à TA et on maintient l'agitation pendant 1 heure. On ajoute 1 ml d'HCl concentré puis on extrait au DCM La phase organique est lavée par une solution de NaOH à 10 % puis par une solution de NaCl. On obtient 15,1 g du composé attendu.

### B) (7-Ethyl-2-méthyl-1H-indol-3-yl)-(2,3-dichlorophényl)méthanone.

On place 14,2 ml de MeMgI 3M dans Et₂O sous argon et on ajoute goutte à goutte, 6,78 g de 7-éthyl-2-méthyl-1*H*-indole dans 40 ml d'Et₂O. Après 30 minutes, on ajoute goutte à goutte, 17,84 g de chlorure de 2,3-dichlorobenzoyle dans 60 ml d'Et₂O et on laisse 2 heures sous agitation. On jette le milieu réactionnel sur une solution saturée de NH₄Cl. On extrait par Et₂O (2 fois) puis on lave la phase organique par une solution de NaOH à 10 %, de l'eau, puis une solution saturée de NaCl. On obtient 5,5 g du composé attendu.

### C) 3-(3-(2,3-dichlorobenzoyl)-7-éthyl-2-méthyl-1H-indol-1-yl)-N,N-diméthyl-1-propanesulfonamide.

On prépare un mélange contenant 1,5 g du composé de l'étape précédente, 3,34 g du composé de l'étape A et 0,15 g de TDA-1 auquel on ajoute 1,7 g de soude pilée et on chauffe à reflux pendant 36 heures. On refroidit puis on verse le milieu réactionnel sur de l'eau. On extrait au toluène puis on lave à l'eau, puis par une solution saturée de NaCl. On sèche, évapore puis le résidu est chromatographié sur silice en éluant par un mélange AcOEt/cyclohexane (1/1 ; v/v). Le produit obtenu cristallise dans un mélange AcOEt/cyclohexane (3/7 ; v/v). On obtient 0,7 g du composé attendu, F = 118°C.
RMN : δ (ppm) : 1,1 : t : 3H ; 2 : mt : 2H ; 2,4 : s : 3H ; 2,8 : s : 6H ; 2,9 : mt : 2H ; 3,2 : mt : 2H ; 4,4 : t : 2H ; 6,8 à 7,2 : m : 3H ; 7,3 à 7,6 : dd + t : 2H ; 7,8 dd : 1H.

En utilisant le procédé A, on prépare les intermédiaires de formule (IV) décrits ci-après :

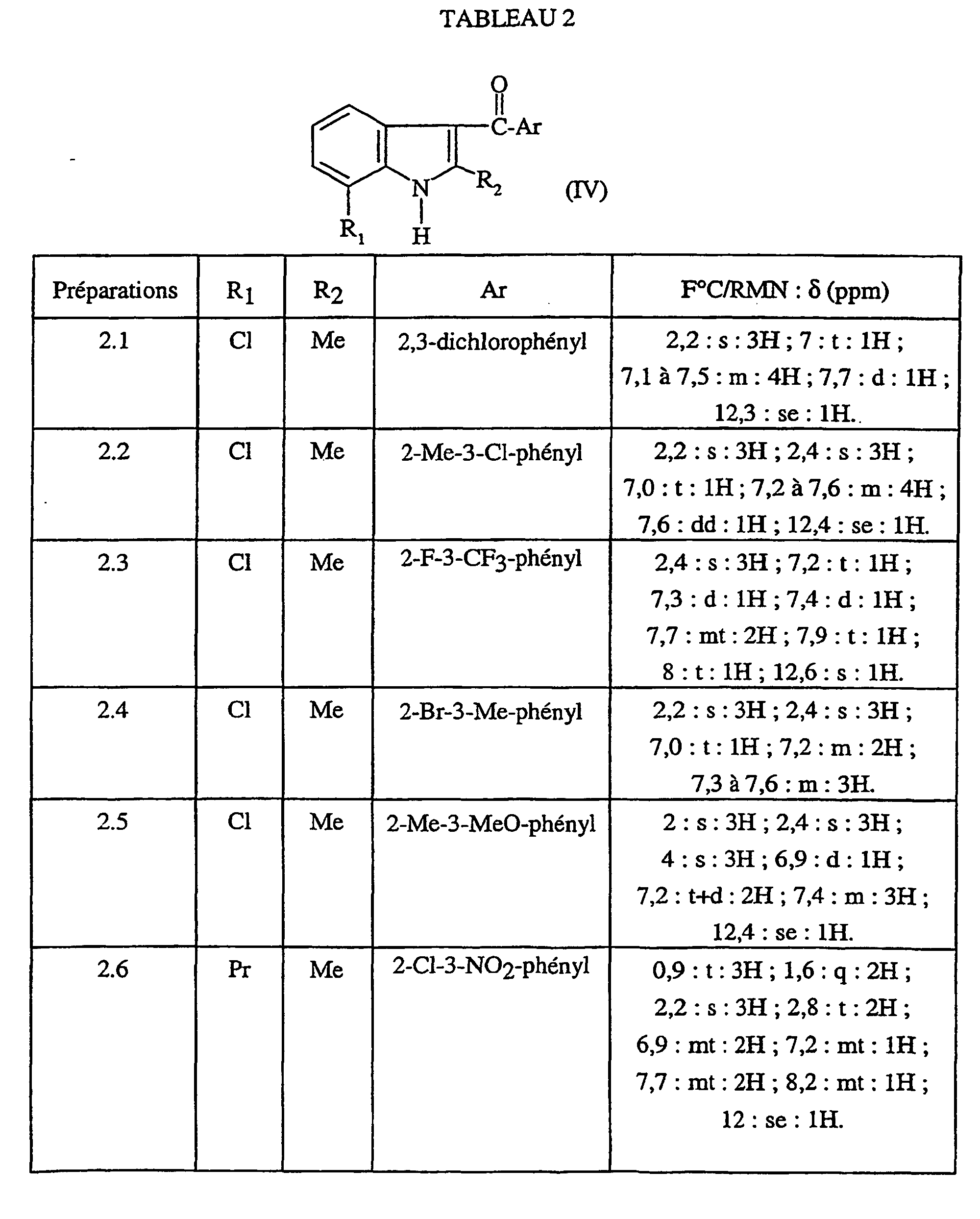

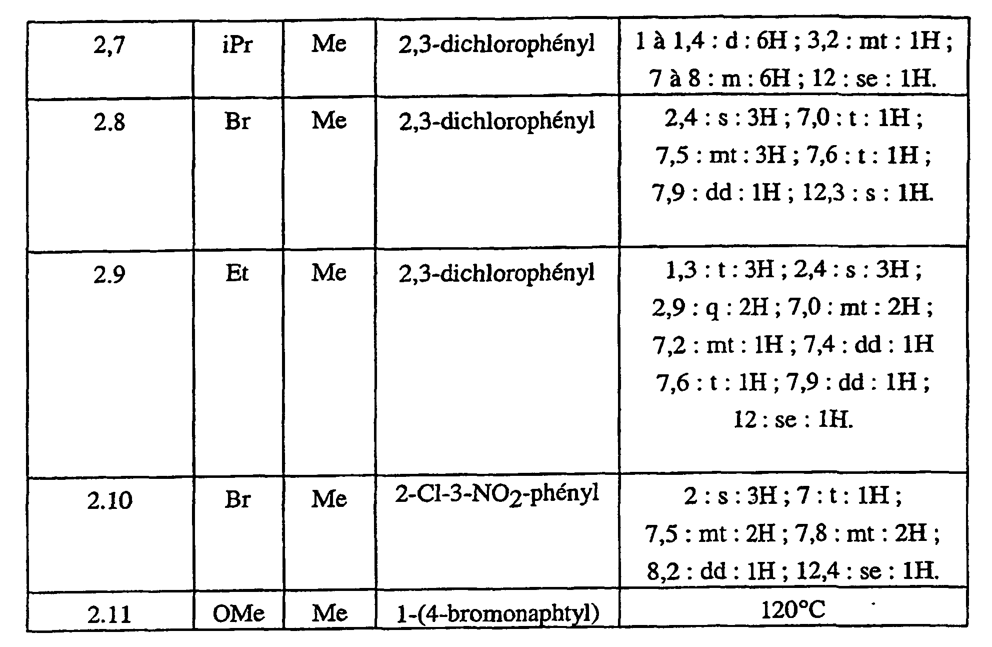

En utilisant le procédé B ou le procédé B₁, on prépare les intermédiaires de formule (XII) décrits ci-après :

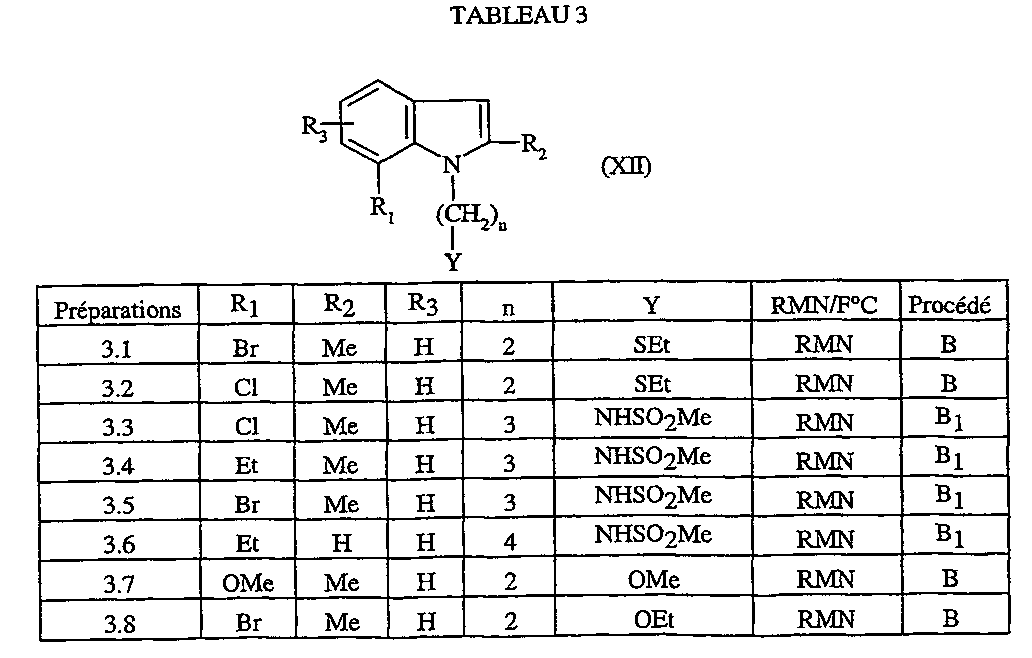

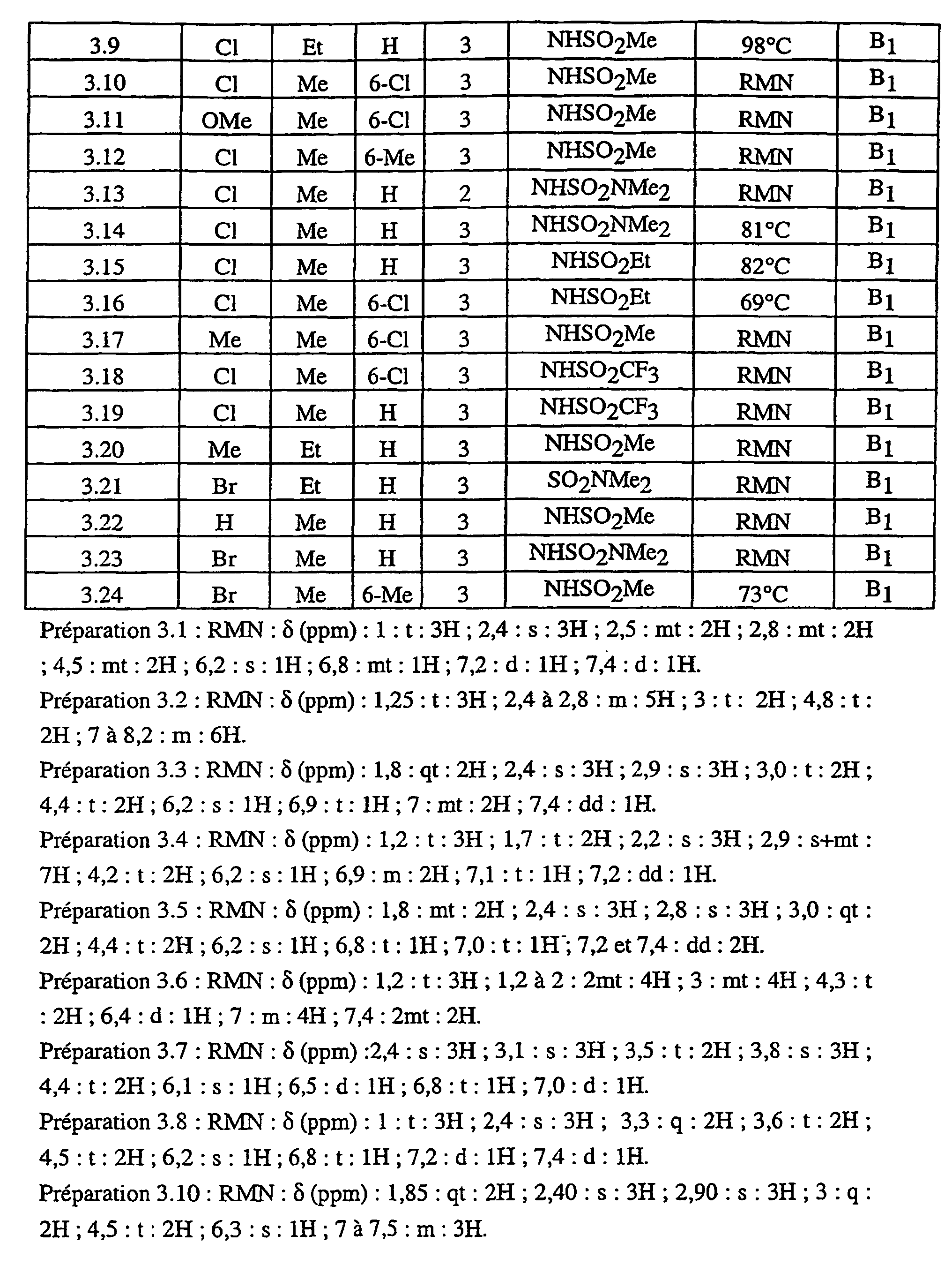

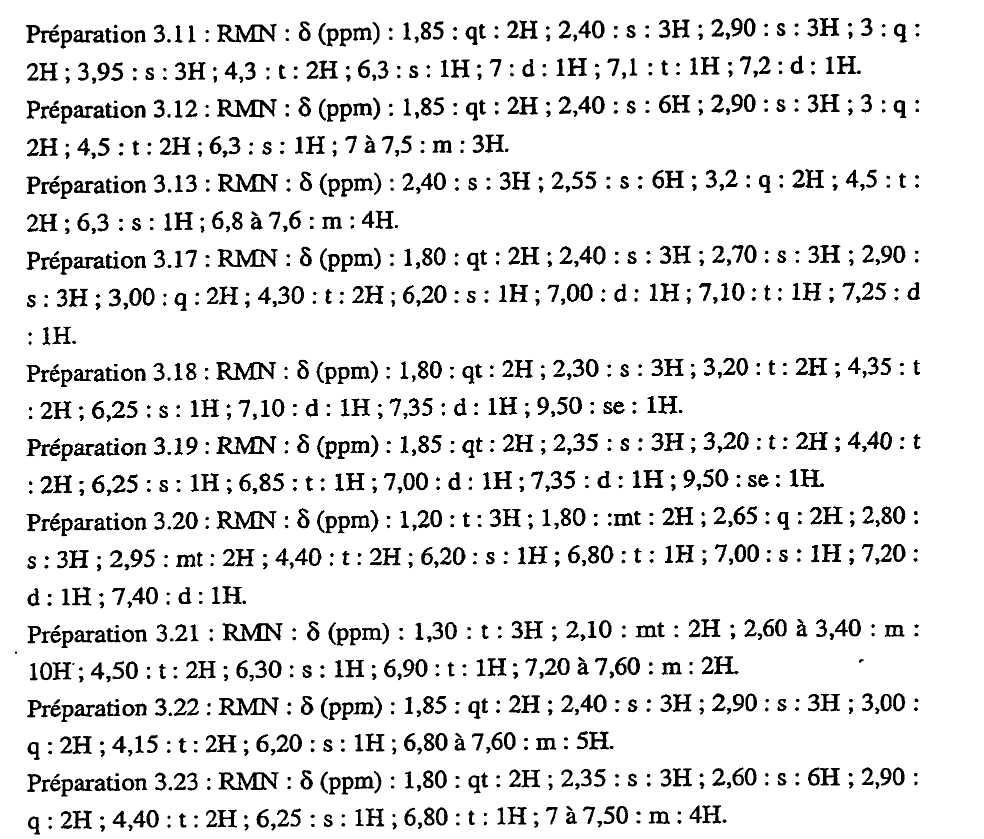

En opérant selon l'un des procédés décrits, on a préparé les composés selon l'invention rassemblés dans le tableau ci-après :

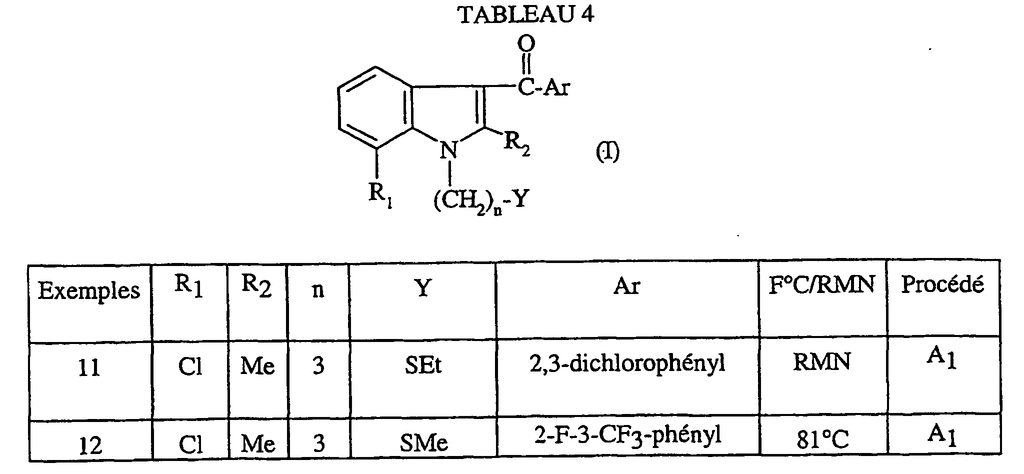

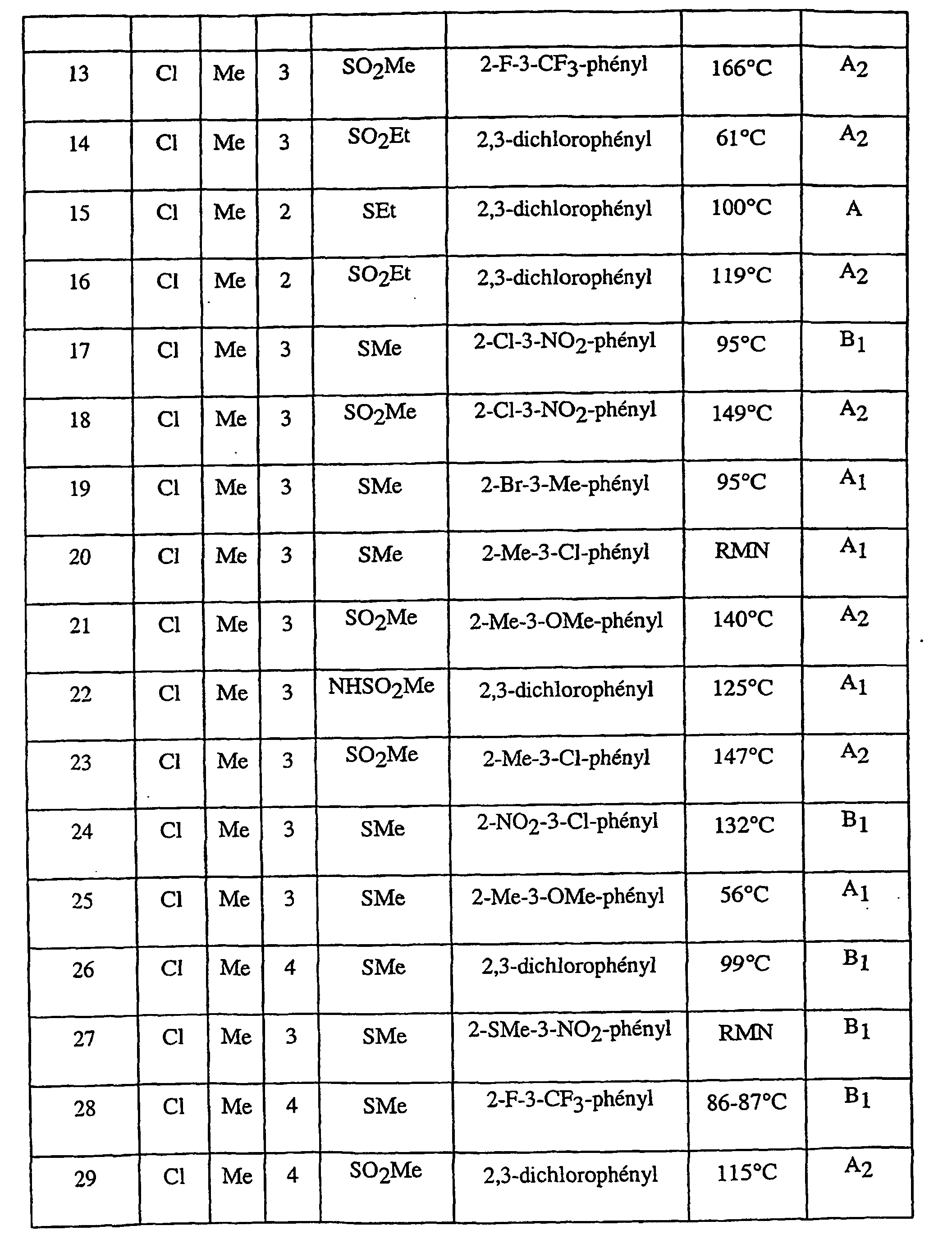

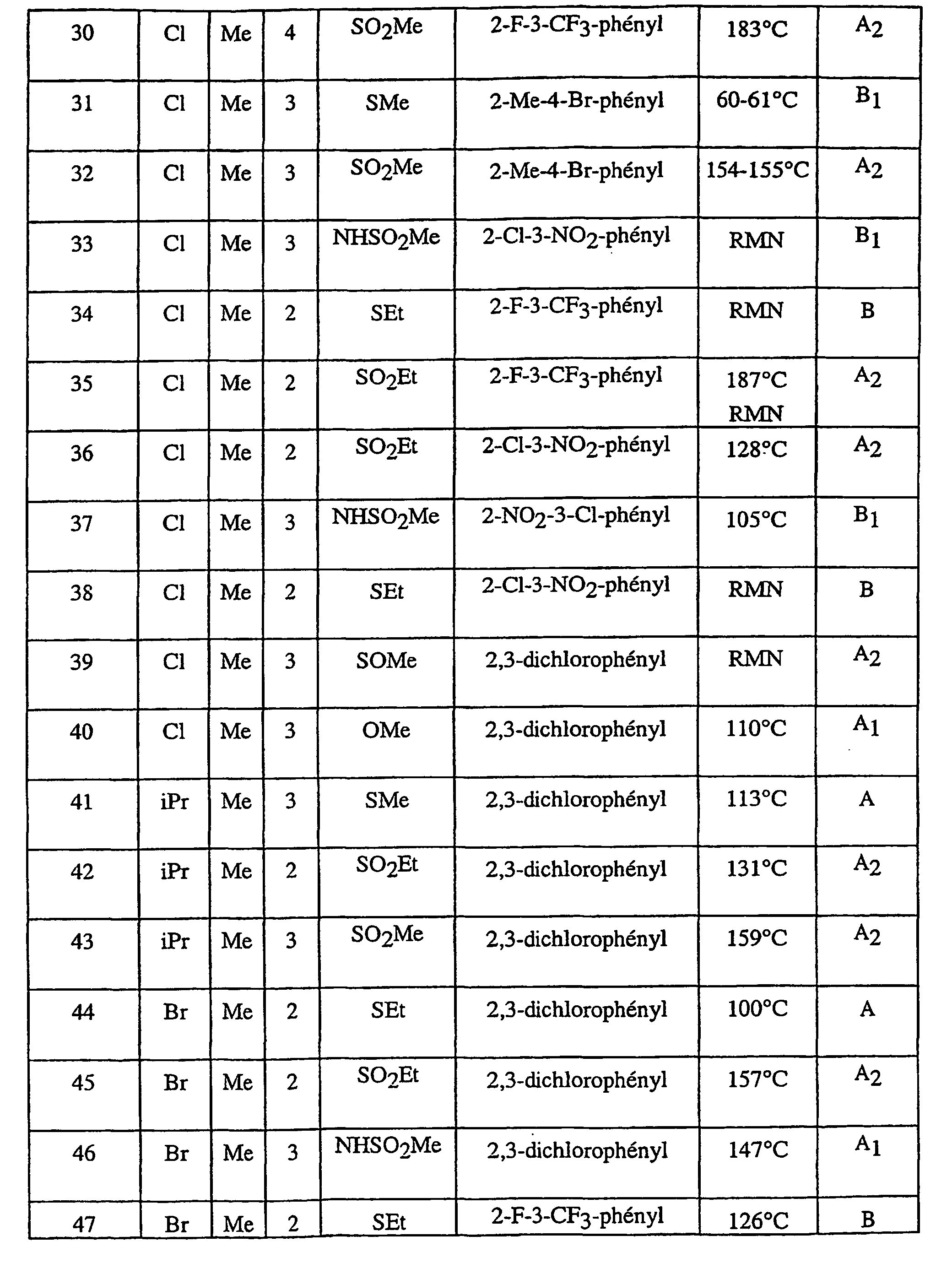

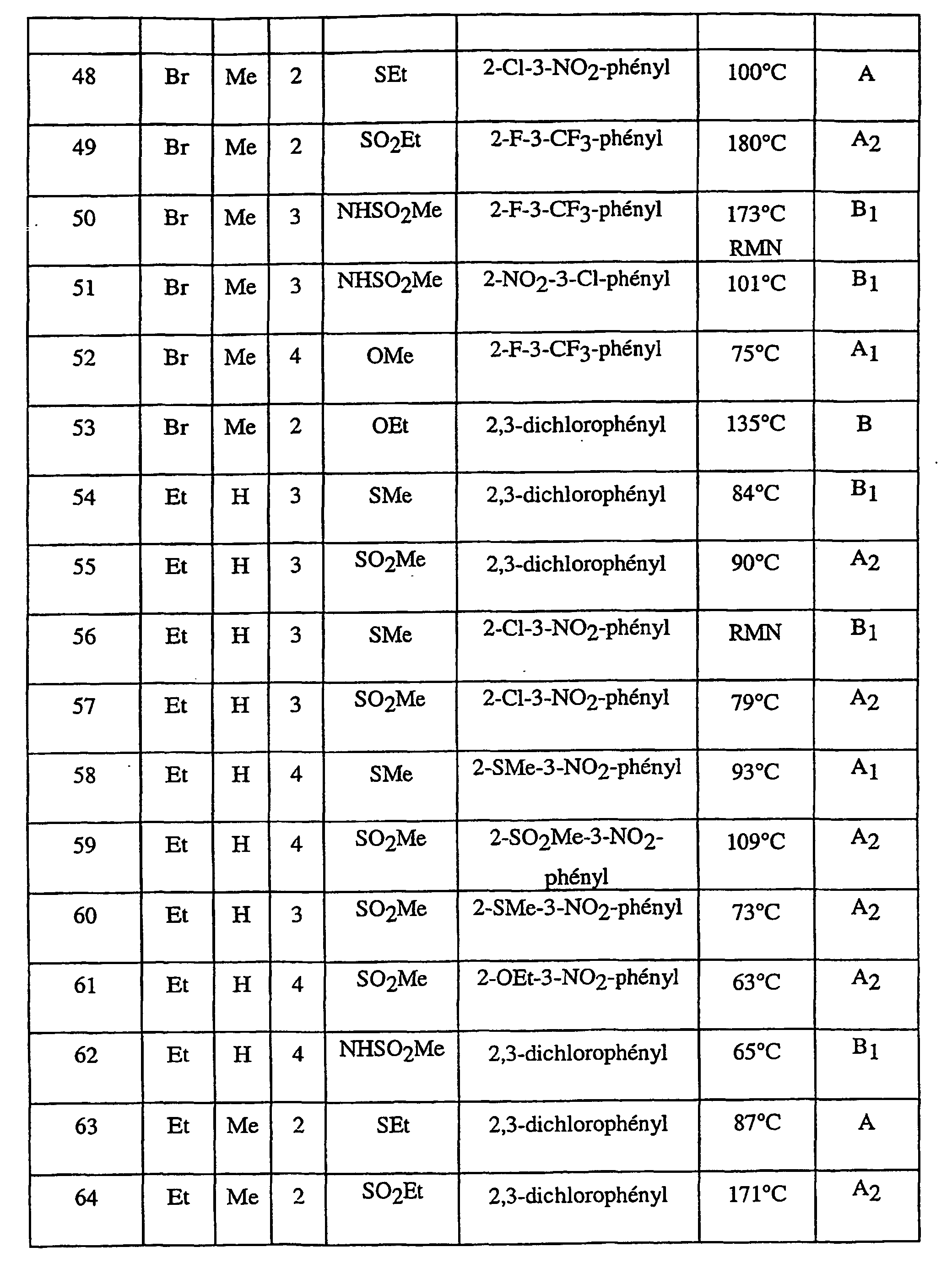

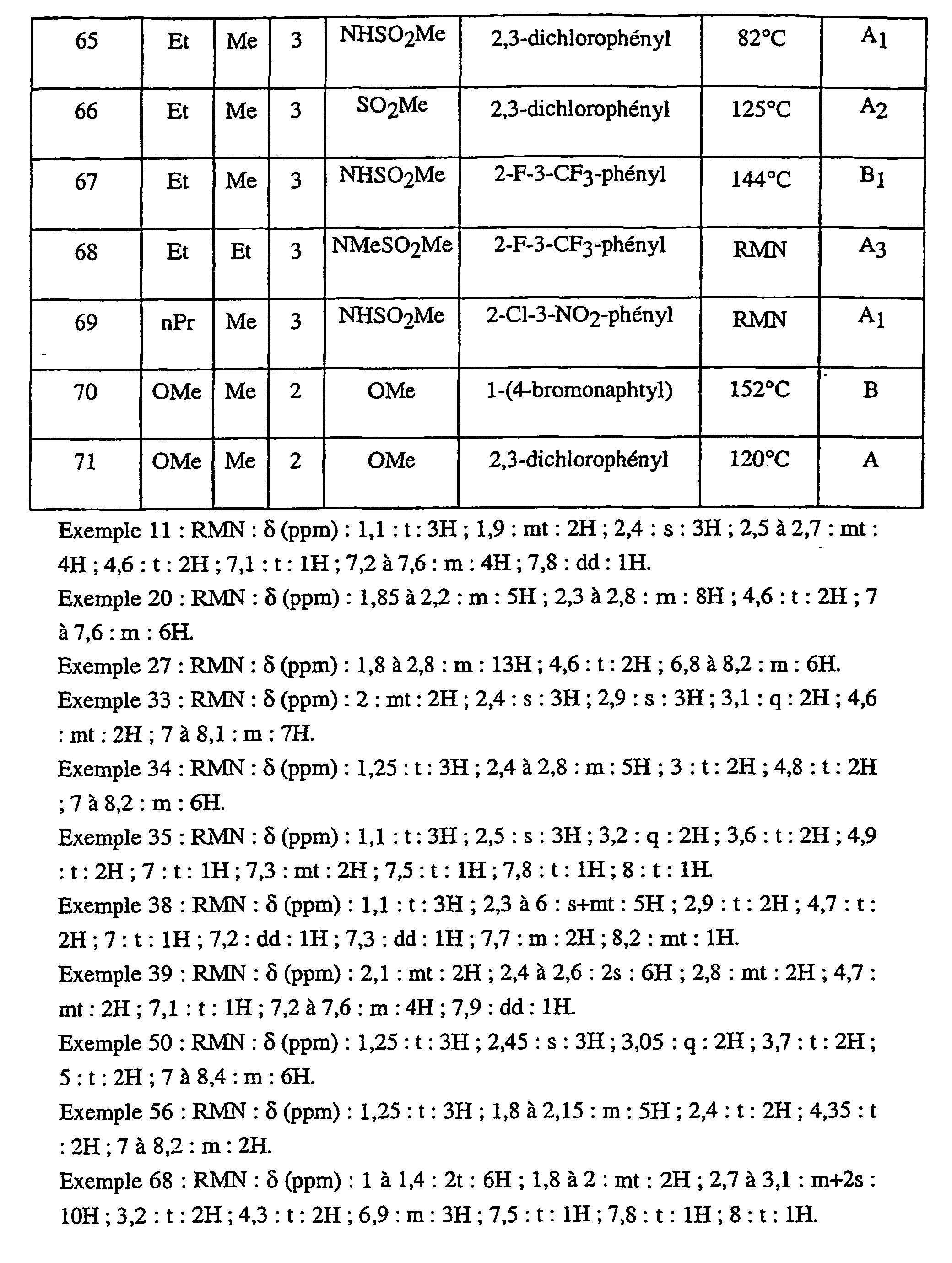

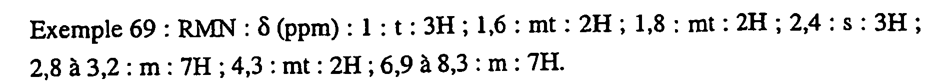

### EXEMPLE 72 (procédé B)

N-(3-(6,7-dichloro-3-[2-fluoro-3-(trifluorométhyl)benzoyl]-2-méthyl-1*H*-indol-1-yl)propyl)méthanesulfonamide.
I : R₁ =R₃ =Cl, R₂ = Me, R'₃ = H, Ar = 2F-3-CF₃-phényle, Y = -NHSO₂Me, A = (CH₂)ₙ, n = 3.

### A) 6,7-Dichloro-1-(3-chloropropyl)-2-méthyl-1H-indole.

Sous azote, on introduit 7,84 g de soude pilée, 190 ml de toluène, 7 g de 6,7-dichloro-2-méthyl-1*H*-indole, 85 ml de toluène et 0,7 g de tétrabutylammonium hydrogénosulfate. On chauffe à reflux pendant 30 minutes puis on ajoute 14 ml de 1-bromo-3-chloropropane et on maintient le reflux pendant 2 heures. On verse le milieu réactionnel sur de l'eau, on lave la phase aqueuse par du toluène. On extrait au toluène puis on lave à l'eau et par une solution saturée de NaCl,. On sèche et évapore pour obtenir 11,3 g du composé attendu.

### B) 6,7-Dichloro-1-(3-iodopropyl)-2-méthyl-1H-indole.

On introduit 11,3 g du composé de l'étape précédente dans 520 ml d'acétonitrile et 43 g de NaI puis on chauffe au reflux pendant 3 jours. On verse le milieu réactionnel sur de l'eau, on le dilue avec du toluène puis on lave 2 fois la phase aqueuse avec du toluène. Les phases organiques sont réunies puis on lave à l'eau puis par une solution saturée de NaCl. On sèche et concentre pour obtenir 13,93 g du composé attendu.

### C) N-[3-(6,7-dichloro-2-méthyl-1H-indol-1-yl)propyl]méthanesulfonamide.

Sous azote, on introduit 6,04 g de NaH à 60 % dans 400 ml de DMF anhydre. On refroidit à 5°C puis on ajoute 14,35 g de méthanesulfonamide dans 200 ml de DMF anhydre. Après 10 minutes sous agitation à 5°C, on ajoute 13,9 g du composé obtenu à l'étape précédente dans 200 ml de DMF anhydre et on laisse revenir à TA. Après 3 heures sous agitation, on verse le milieu réactionnel sur de l'eau puis on dilue par du DCM. On lave 3 fois la phase aqueuse avec du DCM puis on réunit les phases organiques. On lave à l'eau et par une solution saturée de NaCl. On sèche et concentre puis le résidu est chromatographié sur silice en éluant par un mélange cyclohexane/AcOEt (50/50 ; v/v). On obtient 7,43 g du composé attendu.
RMN : δ (ppm) : 1,80 : mt : 2H ; 2,35 : s : 3H ; 2,60 : s : 6H ; 2,90 : mt : 2H ; 4,40 : t : 2H ; 6,30 : s : 1H ; 7,10 : d : 1H ; 7,20 : mt : 1H ; 7,35 : d : 1H.

### D) N-(3-(6,7-dichloro-3-[2-fluoro-3-(trifluorométhyl)benzoyl]-2-méthyl-1H-indol-1-yl)propyl)méthanesulfonamide

On introduit 1 g du composé de l'étape précédente et 1,35 g de chlorure de 2-fluoro-3-(trifluorométhyl)benzoyle dans 120 ml de DCM. On refroidit entre -20°C et - 25°C et on ajoute à l'aide d'une seringue 3,3 ml de dichloroéthylaluminium. On laisse revenir à température ambiante et on maintient 3 heures sous agitation. On verse le milieu réactionnel sur de l'eau, on lave 3 fois la phase aqueuse avec du DCM puis on réunit les phases organiques que l'on filtre sur Célite®. On lave par une solution de NaOH à 10 %, par de l'eau, une solution de HCl à 10 % puis une solution saturée de NaCl. On obtient 0,94 g du composé attendu qui cristallise dans l'éther, F = 181°C.

### EXEMPLE 73 (procédé B)

N-(2-(7-chloro-3-(2,3-dichlorobenzoyl)-2-méthyl-1*H*-indol-1-yl)éthyl)-N,N-diméthylsulfamide.
I : R₁ = 7-Cl, R₂ = Me, R₃ = R'₃ = H, Y = NHSO₂Me₂, A = (CH₂)ₙ, n = 2, Ar = 2,3-dichlorophényle.

### A) 3-(7-Chloro-2-méthyl-1H-indol-1-yl)propanenitrile.

Dans 100 ml de dioxane, on mélange 20 g de 7-chloro-2-méthyl-1*H*-indole avec 3 g de triton B et on ajoute lentement 3 g d'acrylonitrile, on rajoute 250 ml de dioxane puis on chauffe 2 heures à 60°C. On évapore le milieu puis on reprend par AcOEt. Le précipité formé étant éliminé, le produit obtenu est utilisé tel quel à l'étape suivante.

### B) Acide 3-(7-chloro-2-méthyl-1H-indol-1-yl)propanoique.

On prépare une solution de 35 g de KOH dans 350 ml d'eau et on ajoute cette solution au mélange contenant le produit de l'étape précédente dans 175 ml d'éthanol. On chauffe le milieu à 80°C puis on verse le milieu réactionnel dans 500 ml de solution aqueuse d'HCl à 10 %. On extrait au DCM puis on lave par une solution saturée de NaCl. On obtient 22,5 g du composé attendu.

### C) 2-(7-Chloro-2-méthyl-1H-indol-1-yl)-1-éthanamine.

Dans 400 ml de *tert*-butanol, on mélange 22,5 g du composé de l'étape précédente, 27,2 g d'azidure de diphénylphosphoryle et 10 g de triéthylamine et on chauffe à 80°C pendant 3 heures. On verse le milieu réactionnel sur 500 ml de solution saturée de NaHCO₃, on ajoute 500 ml d'AcOEt puis on décante. On évapore la phase organique puis on reprend par un mélange de 10 ml d'HCl concentré dans 500 ml d'éthanol et on chauffe à reflux pendant 6 heures. On basifie à pH 14 par addition de soude à 30 % puis on extrait par AcOEt et on lave par une solution saturée de NaCl. On obtient 2,61 g du composé attendu.

### D) N'-(2-(7-chloro-2-méthyl-1H-indol-1-yl)éthyl)-N,N-diméthylsulfamide.

Sous azote, on met en suspension 2,63 g du composé de l'étape précédente dans 20 ml de CH₃CN et 2 ml de triéthylamine. On prépare une solution de 2 g de chlorure de diméthylsulfamoyle dans 20 ml d'eau et on coule cette solution sur le milieu réactionnel refroidi à -50°C. On laisse revenir à TA puis on chauffe à 50°C pendant 3 heures. On jette le milieu réactionnel sur une solution saturée de NH₄Cl puis on extrait à l'acétonitrile. On sèche et évapore puis on chromatographie le résidu sur silice en éluant par un mélange AcOEt/cyclohexane (1/1 ; v/v). On obtient 0,86 g du composé attendu.
RMN : δ (ppm) : 2,40 : s : 3H ; 2,55 : s : 6H ; 3,2 : q : 2H ; 4,5 : t : 2H ; 6,3 : s : 1H ; 6,8 à 7,6 : m : 4H.

### E) N-(2-(7-chloro-3-(2,3-dichlorobenzoyl)-2-méthyl-1H-indol-1-yl)éthyl)-N,N-diméthylsulfamide.

Sous azote, on introduit 0,86 g du composé de l'étape précédente et 0,75 g de chlorure de 2,3-dichlorobenzoyle dans 50 ml de DCM. On refroidit à -50°C et, à cette température, on ajoute 4,8 ml de dichloroéthyl aluminium 1,8 M dans le toluène. On laisse revenir à TA et on maintient 3 heures sous agitation. On jette le milieu réactionnel sur NH₄Cl puis on extrait au DCM. On lave par une solution d'HCl à 10 %, par une solution de NaOH à 10 % puis par une solution saturée de NaCl. On sèche et évapore puis le résidu est chromatographié sur silice en éluant par le mélange AcOEt/cyclohexane (1/1 ; v/v) On obtient 0,49 g du composé attendu, F = 75°C.

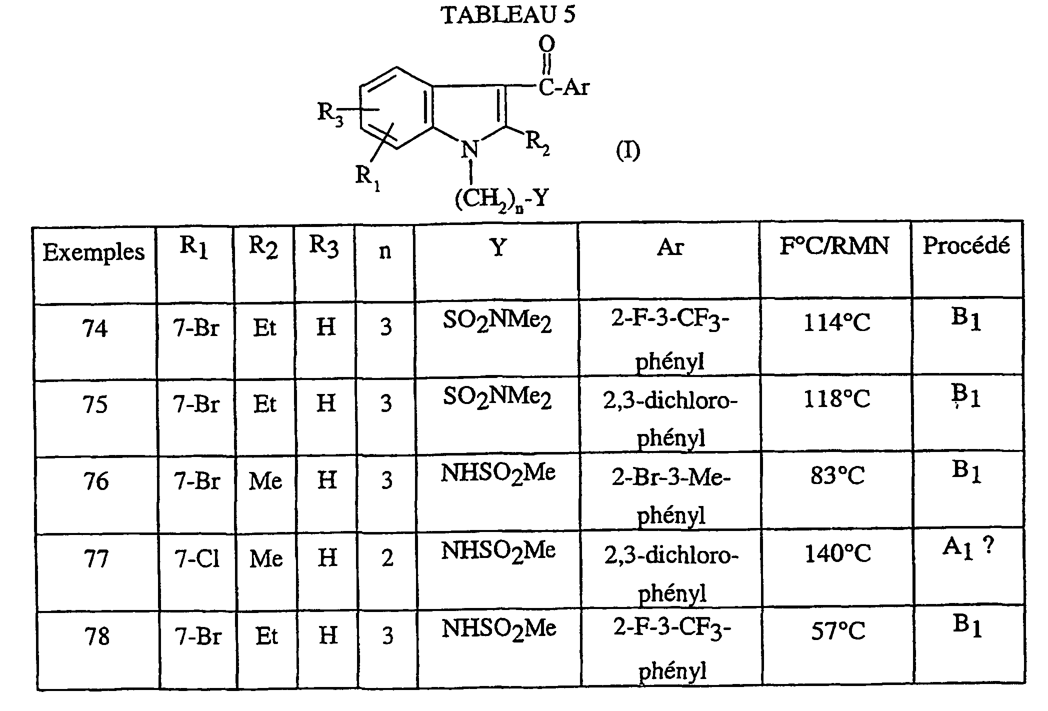

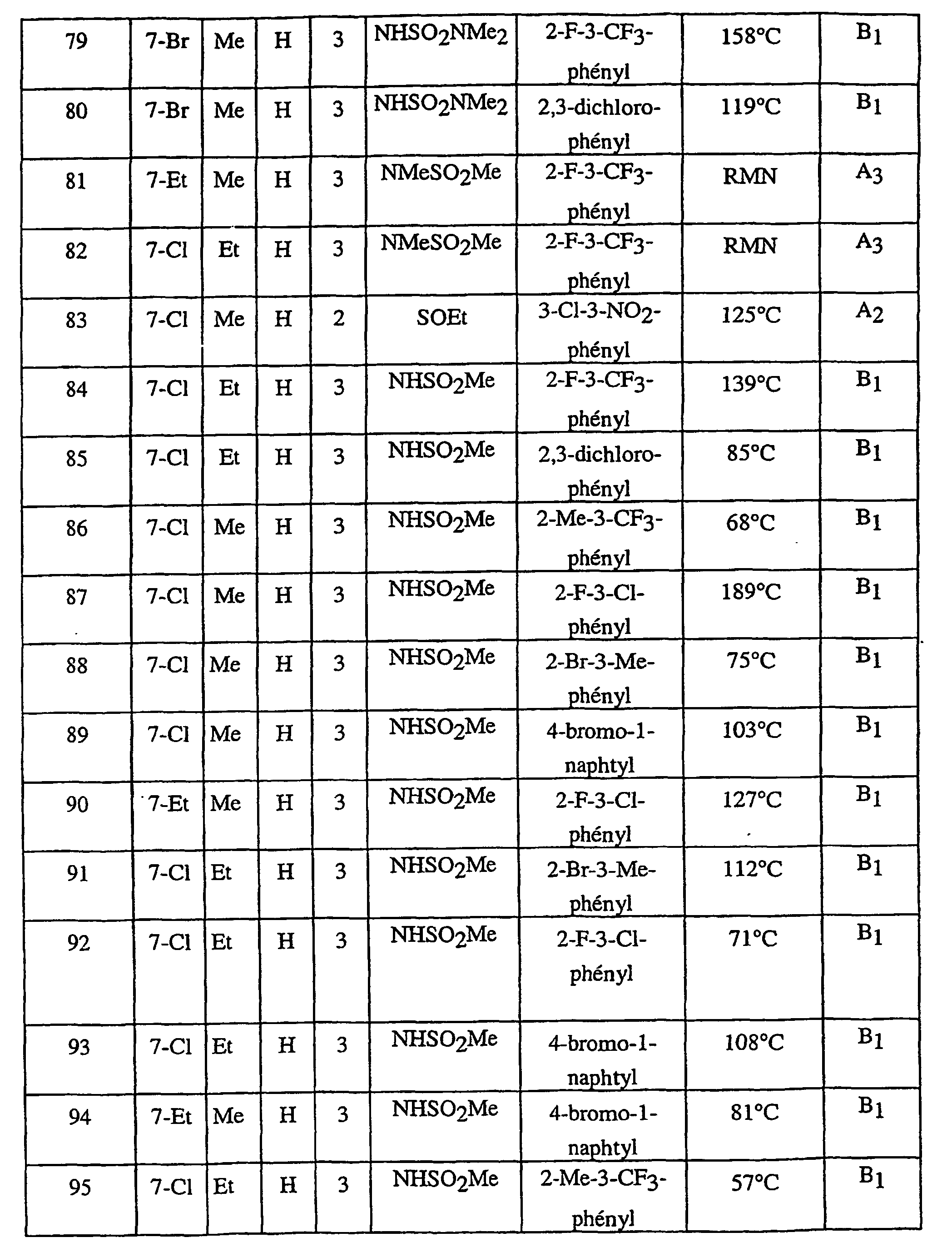

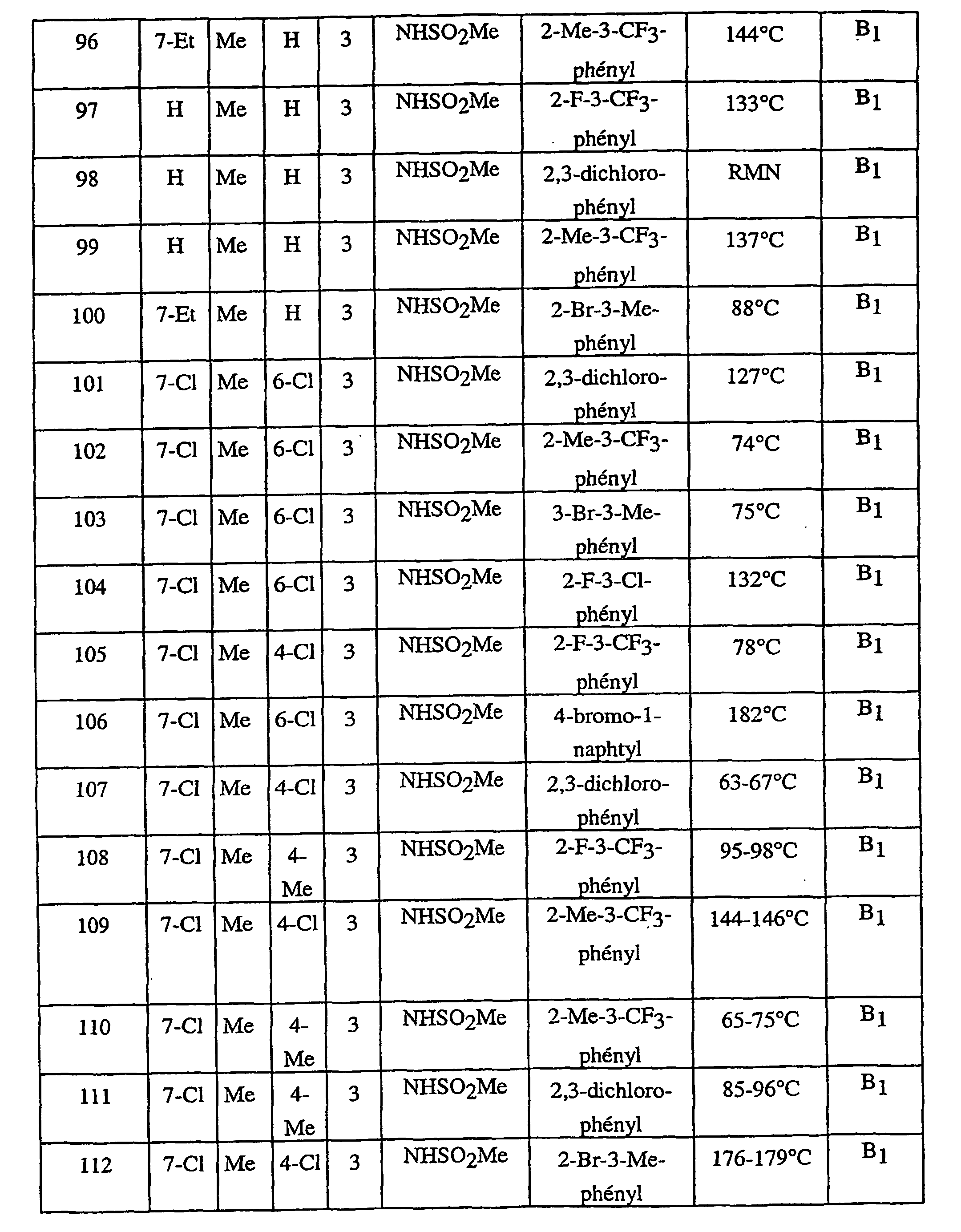

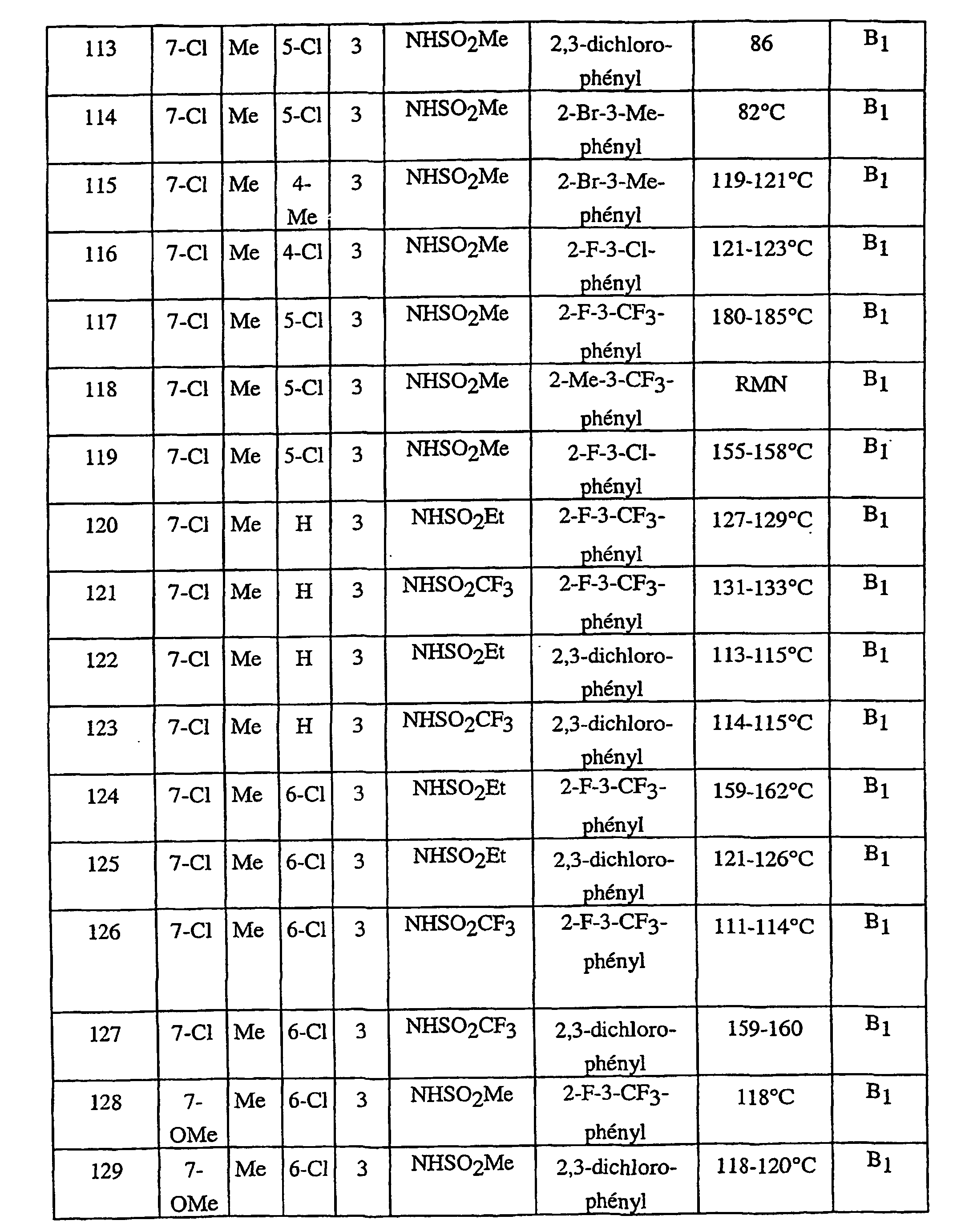

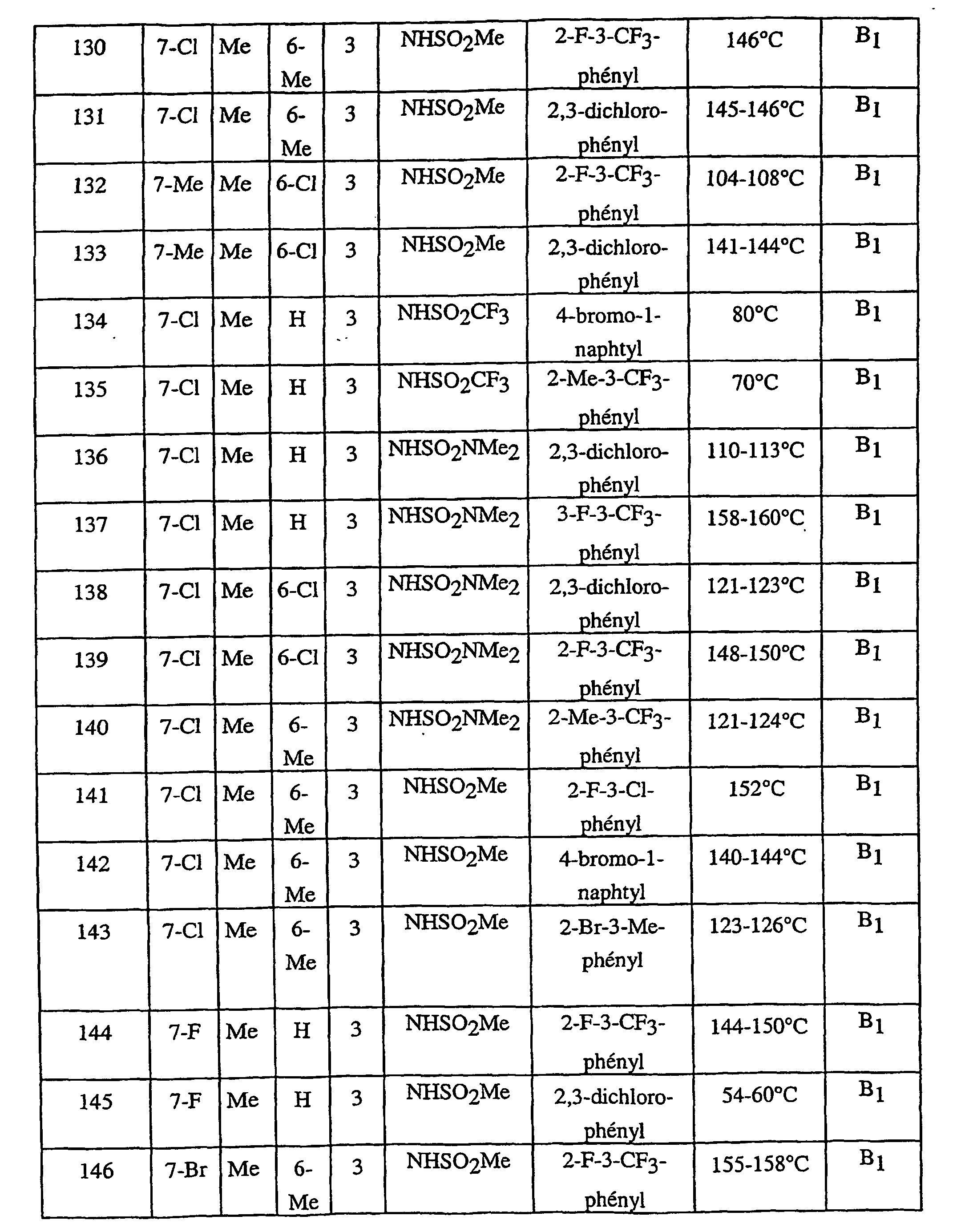

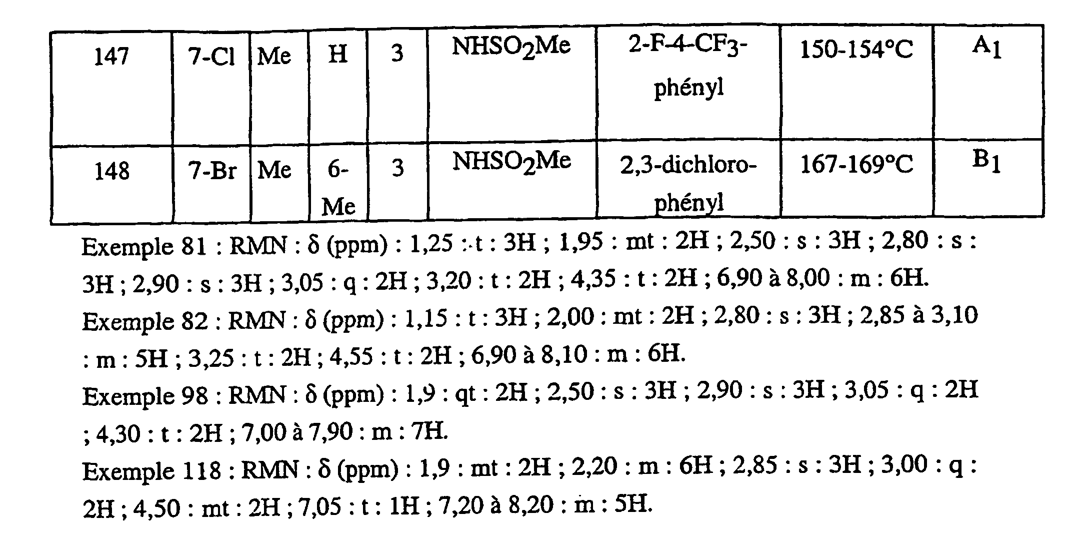

## Revendications

1. Un composé de formule : 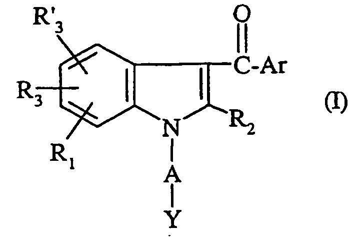 dans laquelle :
- Ar représente:
a) un phényle mono, di ou trisubstitué par un ou plusieurs groupes choisis parmi :
un atome d'halogène, un (C₁-C₄)alkyle, un trifluorométhyle, un amino, un nitro, un hydroxyle, un (C₁-C₄)alcoxy, un (C₁-C₄)alkylsulfanyle, un (C₁-C₄) alkylsulfonyle ;
b) un naphtyle non substitué ou substitué une ou deux fois par un atome d'halogène, un (C₁-C₄)alkyle, un trifluorométhyle ;
- A représente un radical alkylène en C₂-C₆ ;
- Y représente un groupe choisi parmi SR₄, SOR₄, SO₂R₄, SO₂NR₅R₆, N(R₇)SO₂R₄, OR₄, NR₇SO₂NR₅R₆ ;
- R₁, R₃ et R'₃ représentent chacun indépendamment l'un de l'autre l'hydrogène, un hydroxyle, un atome d'halogène, un (C₁-C₄)alkyle, un trifluorométhyle, un (C₁-C₄)alcoxy ;
- R₂ représente l'hydrogène ou un (C₁-C₄)alkyle ;
- R₄ représente un (C₁-C₄)alkyle, un trifluorométhyle ;
- R₅ et R₆ représentent chacun indépendamment l'hydrogène ou un (C₁-C₄) alkyle ;
- R₇ représente l'hydrogène ou un (C₁-C₄)alkyle ;
ainsi que ses sels éventuels et/ou ses solvats.

2. Un composé selon la revendication 1 de formule : 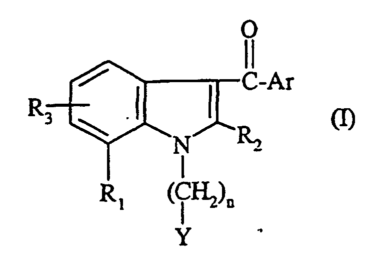 dans laquelle :
- Ar représente:
a) un phényle mono, di ou trisubstitué par un ou plusieurs groupes choisis parmi :
un atome d'halogène, un (C₁-C₄)alkyle, un trifluorométhyle, un amino, un nitro, un (C₁-C₄)alcoxy, un (C₁-C₄)alkylsulfanyle, un (C₁-C₄)alkylsulfonyle ;
b) un naphtyle non substitué ou substitué une ou deux fois par un atome d'halogène, un (C₁-C₄)alkyle, un trifluorométhyle ;
- n représente 2, 3 ou 4 ;
- Y représente un groupe choisi parmi SR₄, SOR₄, SO₂R₄, SO₂NR₅R₆, N(R₇)SO₂R₄, OR₄ ;
- R₁ représente un atome d'halogène, un (C₁-C₄)alkyle, un trifluorométhyle, un (C₁-C₄)alcoxy ;
- R₂ représente l'hydrogène ou un (C₁-C₄)alkyle ;
- R₃ représente l'hydrogène, un (C₁-C₄)alkyle ou un halogène ;
- R₄ représente un (C₁-C₄)alkyle ;
- R₅ et R₆ représentent chacun indépendamment l'hydrogène ou un (C₁-C₄) alkyle ;
- R₇ représente l'hydrogène ou un (C₁-C₄)alkyle ;
ainsi que ses sels éventuels et/ou ses solvats.

3. Un composé selon la revendication 1 de formule (I) dans laquelle R₁ est en position 7 du noyau indole et représente un méthyle, un atome de chlore ou de brome.

4. Un composé selon la revendication 1 de formule (I) dans laquelle R₂ représente un (C₁-C₄)alkyle.

5. Un composé selon la revendication 1 de formule (I) dans laquelle R₃ est l'hydrogène ou R₃ est en position 6 du noyau indole et représente, soit un atome de chlore, soit un méthyle.

6. Un composé selon la revendication 1 de formule (I) dans laquelle R'₃ est l'hydrogène.

7. Un composé selon la revendication 1 de formule (I) dans laquelle Ar représente un phényle mono ou disubstitué par un atome d'halogène, un méthyle, un trifluorométhyle, un méthoxy, un méthylsulfanyle, un méthylsulfonyle.

8. Un composé selon la revendication 1 de formule (I) dans laquelle Y représente SO₂R₄ ou NH SO₂R₄.

9. Un composé selon la revendication 1 de formule (I) dans laquelle :
- Ar représente un phényle mono ou disubstitué par un atome d'halogène, un méthyle, un trifluorométhyle, un méthoxy, un méthylsulfanyle, un méthylsulfonyle ;
- A représente un groupe (CH₂)ₙ ;
- n représente 2, 3 ou 4 ;
- Y représente SO₂R₄ ou NHSO₂R₄ ;
- R₁ représente un méthyle, un atome de chlore ou de brome, en position 7 du noyau indole ;
- R₂ représente un méthyle ;
- R₃ est l'hydrogène ou R₃ représente soit un atome de chlore, soit un méthyle, en position 6 du noyau indole ;
- R'₃ est l'hydrogène ;
- R₄ représente un méthyle ou un éthyle ;
ainsi que ses sels éventuels et/ou ses solvats.

10. Procédé de préparation d'un composé de formule (I) selon la revendication 1, de ses sels éventuels et/ou ses solvats, **caractérisé en ce que** :
a) on traite un indole de formule : 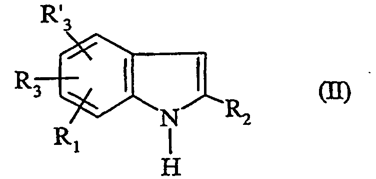 dans laquelle R₁, R₂, R₃ et R'₃ sont tels que définis à la revendication 1 pour un composé de formule (I), par un halogénure de méthylmagnésium et par un halogénure d'acide de formule ArCOHal (III), dans laquelle Ar est tel que défini pour le composé de formule (I) et Hal représente un atome d'halogène ;
b) on traite le composé ainsi obtenu de formule : 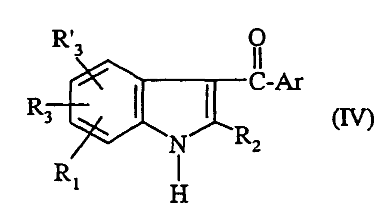 par un halogénure de formule Hal-A-Y (V) dans laquelle -A- et Y sont tels que définis pour un composé de formule (I) à la revendication 1 et Hal représente un atome d'halogène, en présence d'une base.

11. Procédé selon la revendication 10, **caractérisé en ce que** l'étape b) est modifiée de la façon suivante :
b1) on traite le composé obtenu à l'étape a) de formule : 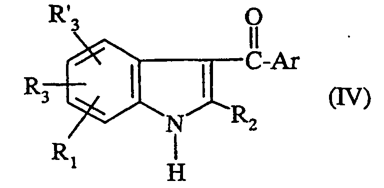 par un composé de formule Z-A-Cl (VI), dans laquelle Z représente soit un groupe hydroxyle soit un atome d'halogène, et -A- est tel que défini pour (I) à la revendication 1 ;
b2) éventuellement, on traite le composé ainsi obtenu de formule : 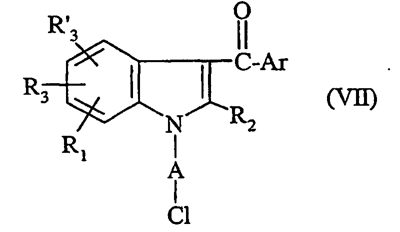 par l'iodure de sodium ;
b3) on traite le composé ainsi obtenu à l'étape b1 de formule (VII) ou à l'étape b2) de formule : 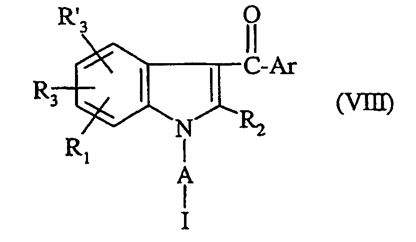 par un anion Y^{⊖}, Y étant tel que défini pour un composé de formule (I) à la revendication 1.

12. Procédé selon la revendication 10 pour préparer un composé de formule (I) dans laquelle Y représente un groupe SOR₄ ou un groupe SO₂R₄, à partir d'un composé de formule (I) dans laquelle Y représente un groupe SR₄, **caractérisé en ce que**, on effectue l'étape supplémentaire suivante :
c1) on traite le composé obtenu de formule : 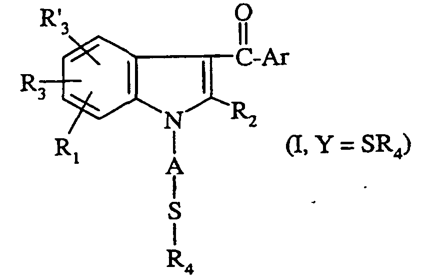 par un agent oxydant.

13. Procédé selon la revendication 10 pour préparer un composé de formule (I) dans laquelle Y représente un groupe N(R₇) SO₂R₄ avec R₇ différent de H, à partir d'un composé de formule (I) dans laquelle Y représente un groupe NHSO₂R₄, **caractérisé en ce que**, on effectue l'étape supplémentaire suivante :
c2) on traite le composé obtenu de formule : 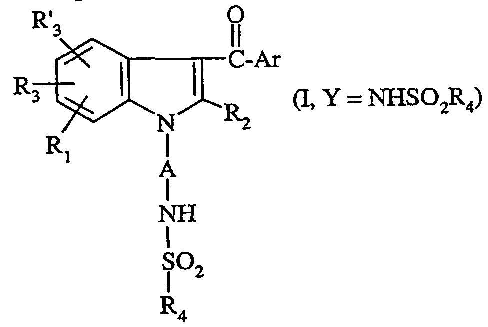 par un agent alkylant, en présence d'une base.

14. Procédé selon la revendication 10 pour préparer un composé de formule (I) dans laquelle Y représente un groupe SO₂NR₅R₆ à partir d'un composé de formule (I) dans laquelle Y représente un groupe SO₂NHR₅, **caractérisé en ce que**, on effectue l'étape supplémentaire suivante :
c3) on traite le composé obtenu de formule : 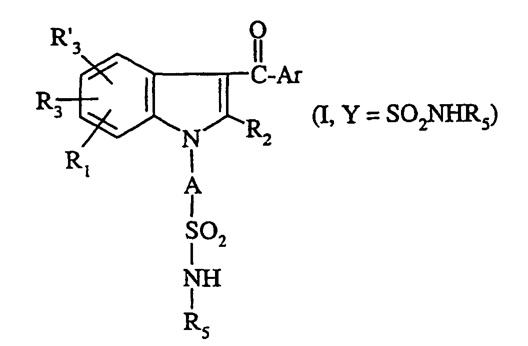 par un agent alkylant, en présence d'une base.

15. Procédé selon la revendication 10 pour préparer un composé de formule (I) dans laquelle Y représente un groupe NR₇SO₂R₄ ou un groupe NR₇SO₂NR₅R₆, **caractérisé en ce que** :
b4) on transforme le composé de formule : 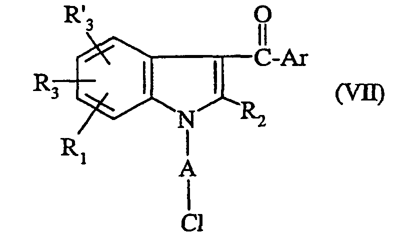 en un composé de formule : 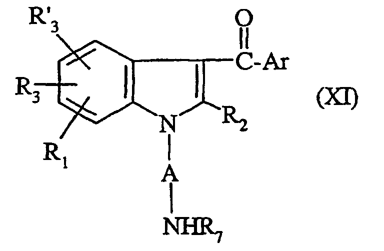 dans laquelle R₇ est tel que défini pour (I) ;
c4) on traite par un halogénure de formule HalSO₂R₄ ou respectivement HalSO₂NR₅R₆ dans laquelle R₄, R₅ et R₆ ont les significations données à la revendication 1 pour un composé de formule (I).

16. Procédé de préparation d'un composé de formule (I) selon la revendication 1, de ses sels et/ou solvats, **caractérisé en ce que** :
i) on traite un indole de formule : 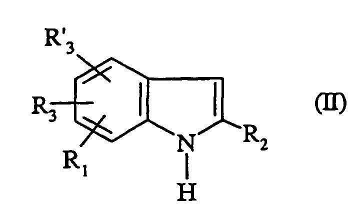 dans laquelle R₁, R₂, R₃ et R'₃ sont tels que définis pour le composé de formule (I) par un halogénure de formule Hal-A-Y (V) dans laquelle -A- et Y sont tels que définis pour un composé de formule (I) à la revendication 1 et Hal représente un atome d'halogène, de préférence le brome, en présence d'une base ;
ii) on traite le composé ainsi obtenu de formule : 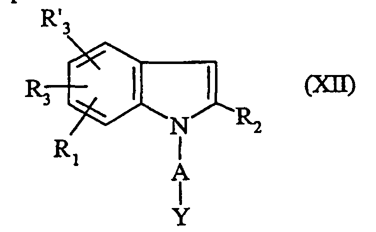 par un halogénure d'acide de formule ArCOHal (III) dans laquelle Ar est tel que défini pour le composé de formule (I) à la revendication 1 et Hal est un atome d'halogène.

17. Procédé selon la revendication 16, **caractérisée en ce que** :
i1) on traite un indole de formule : 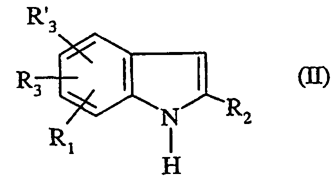 dans laquelle R₁, R₂, R₃ et R'₃ sont tels que définis pour le composé de formule (I), avec un composé de formule Z-A-Cl (VI) dans laquelle -A- est tel que défini pour le composé de formule (I) à la revendication 1 et Z représente un groupe hydroxyle ou un atome d'halogène ;
i2) éventuellement, on traite le composé ainsi obtenu de formule : 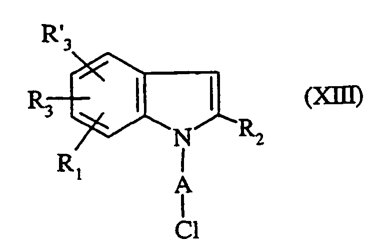 par l'iodure de sodium ;
i3) on traite le composé ainsi obtenu à l'étape i1) ou à l'étape i2) de formule : 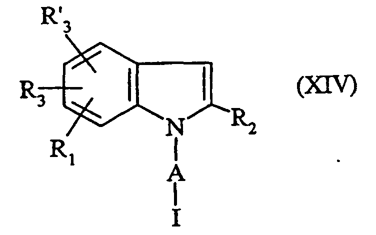 par un anion de formule Y⁻, Y étant tel que défini pour un composé de formule (I) à la revendication 1 ;
ii) on traite le composé ainsi obtenu de formule : 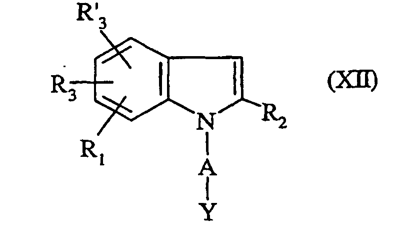 par un halogénure d'acide de formule ArCOHal (III) dans laquelle Ar est tel que défini pour le composé de formule (I) à la revendication 1 et Hal est un atome d'halogène.

18. Un composé de formule : 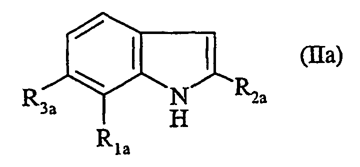 dans laquelle :
- R₁ₐ représente un atome de chlore ou de brome ou un méthyle ;
- R₂ₐ représente un (C₁-C₄)alkyle ;
- R₃ₐ représente l'hydrogène, un atome de chlore ou de brome, ou un méthyle.

19. Un composé de formule : 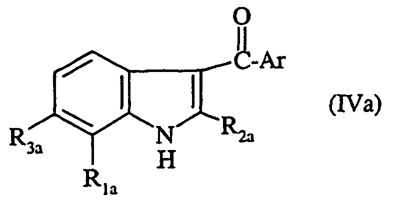 dans laquelle :
- R₁ₐ représente un atome de chlore ou de brome ou un méthyle ;
- R₂ₐ représente un (C₁-C₄)alkyle ;
- R₃ₐ représente l'hydrogène, un atome de chlore ou de brome, ou un méthyle ;
- Ar est tel que défini pour les composés de formule (I).

20. Un composé de formule : 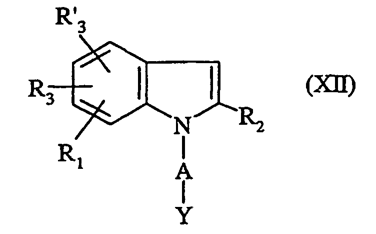 dans laquelle R₁, R₂, R₃, R'₃ et Y sont tels que définis pour (I) à la revendication 1.

21. Composition pharmaceutique contenant en tant que principe actif un composé selon l'une quelconque des revendications 1 à 9.

22. Composition pharmaceutique selon la revendication 21, contenant de 0,1 à 1000 mg de principe actif, sous forme d'unité de dosage dans laquelle le principe actif est mélangé à au moins un excipient pharmaceutique.

23. Médicament **caractérisé en ce qu'**il est constitué d'un composé selon l'une quelconque des revendications 1 à 9.

24. Utilisation d'un composé selon l'une quelconque des revendications 1 à 9 pour la préparation de médicaments destinés à combattre toute pathologie dans laquelle les récepteurs aux cannabinoïdes CB₂ sont impliqués.

## Patentansprüche

1. Verbindung der Formel: 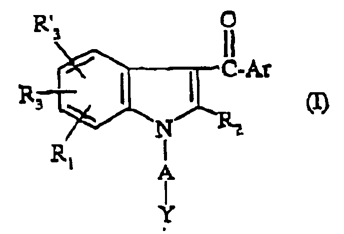 wobei:
- Ar folgendes darstellt:
a) ein Phenyl, das mono-, di- oder trisubstituiert ist mit einer oder mehreren Gruppen, ausgewählt aus: einem Halogenatom, (C₁-C₄)-Alkyl, Trifluormethyl, einer Amino-, einer Nitro-, einer Hydroxylgruppe, (C₁-C₄)-Alkoxy, (C₁-C₄)-Alkylsulfanyl, (C₁-C₄)-Alkylsulfonyl;
b) ein Naphthyl, das nicht substituiert oder ein- oder zweifach substituiert ist mit einem Halogenatom, (C₁-C₄)-Alkyl, Trifluormethyl;
- A einen C₂-C₆-Alkylenrest darstellt;
- Y eine Gruppe darstellt, ausgewählt aus SR₄, SOR₄, SO₂R₄, SO₂NR₅R₆, N(R₇)SO₂R₄, OR₄, NR₇SO₂NR₅R₆;
- R₁, R₃ und R'₃ jeweils unabhängig voneinander Wasserstoff, Hydroxyl, ein Halogenatom, (C₁-C₄)-Alkyl, Trifluormethyl, (C₁-C₄)-Alkoxy darstellen;
- R₂ Wasserstoff oder (C₁-C₄)-Alkyl darstellt;
- R₄ (C₁-C₄)-Alkyl, Trifluormethyl darstellt;
- R₅ und R₆ jeweils unabhängig Wasserstoff oder (C₁-C₄)-Alkyl darstellen;
- R₇ Wasserstoff oder (C₁-C₄)-Alkyl darstellt;
sowie ihre möglichen Salze und/oder ihre Solvate.

2. Verbindung nach Anspruch 1 der Formel 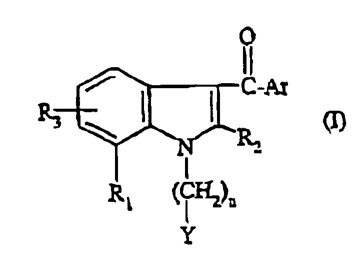 wobei
- Ar folgendes darstellt:
a) ein Phenyl, das mono-, di- oder tri-substituiert ist mit einer oder mehreren Gruppen, ausgewählt aus: einem Halogenatom, (C₁-C₄)-Alkyl, Trifluormethyl, einer Amino-, einer Nitrogruppe, (C₁-C₄)-Alkoxy, (C₁-C₄)-Alkylsulfanyl, (C₁-C₄)-Alkylsulfonyl;
b) ein Naphthyl, das nicht substituiert oder ein- oder zweifach substituiert ist mit einem Halogenatom, (C₁-C₄)-Alkyl, Trifluormethyl;
- n 2, 3 oder 4 darstellt;
- Y eine Gruppe darstellt, ausgewählt aus SR₄, SOR₄, SO₂R₄, SO₂NR₅R₆, N(R₇)SO₂R₄, OR₄;
- R₁ ein Halogenatom, (C₁-C₄)-Alkyl, Trifluormethyl, (C₁-C₄)-Alkoxy darstellt;
- R₂ Wasserstoff oder (C₁-C₄)-Alkyl darstellt;
- R₃ Wasserstoff, (C₁-C₄)-Alkyl, oder Halogen darstellt;
- R₄ (C₁-C₄)-Alkyl darstellt;
- R₅ und R₆ jeweils unabhängig Wasserstoff oder (C₁-C₄)-Alkyl darstellen;
- R₇ Wasserstoff oder (C₁-C₄)-Alkyl darstellt;
sowie ihre möglichen Salze und/oder ihre Solvate.

3. Verbindung nach Anspruch 1 der Formel (I), wobei sich R₁ in Position 7 des Indolkerns befindet und Methyl, ein Chloratom oder ein Bromatom darstellt.

4. Verbindung nach Anspruch 1 der Formel (I), wobei R₂ (C₁-C₄)-Alkyl darstellt.

5. Verbindung nach Anspruch 1 der Formel (I), wobei R₃ Wasserstoff ist oder R₃ sich in Position 6 des Indolkerns befindet und entweder ein Chloratom oder Methyl darstellt.

6. Verbindung nach Anspruch 1 der Formel (I), wobei R'₃ Wasserstoff ist.

7. Verbindung nach Anspruch 1 der Formel (I), wobei Ar ein Phenyl darstellt, das mono- oder disubstituiert ist mit einem Halogenatom, Methyl, Trifluormethyl, Methoxy, Methylsulfanyl, Methylsulfonyl.

8. Verbindung nach Anspruch 1 der Formel (I), wobei Y SO₂R₄ oder NHSO₂R₄ darstellt.

9. Verbindung nach Anspruch 1 der Formel (I), wobei
- Ar Phenyl darstellt, das mono- oder disubstituiert ist mit einem Halogenatom, Methyl, Trifluormethyl, Methoxy, Methylsulfanyl, Methylsulfonyl;
- A eine Gruppe (CH₂)ₙ darstellt;
- n 2,3, oder 4 darstellt;
- Y SO₂R₄ oder NHSO₂R₄ darstellt;
- R₁ Methyl, ein Chlor- oder Bromatom in Position 7 des Indolkerns darstellt;
- R₂ ein Methyl darstellt;
- R₃ Wasserstoff ist oder R₃ entweder ein Chloratom oder Methyl in Position 6 des Indolkerns darstellt;
- R'₃ Wasserstoff ist;
- R₄ Methyl oder Ethyl darstellt;
sowie ihre möglichen Salze und/oder Solvate.

10. Verfahren zur Herstellung einer Verbindung der Formel (I) nach Anspruch 1, ihrer möglichen Salze und/oder ihrer Solvate, **dadurch gekennzeichnet, dass**
a) ein Indol der Formel 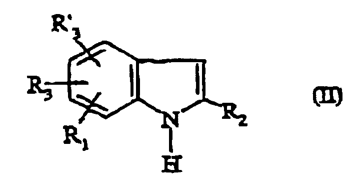 wobei R₁, R₂, R₃ und R'₃ wie nach Anspruch 1 für eine Verbindung der Formel (I) definiert sind, mit einem Methylmagnesiumhelogenid und mit einem Säurehalogenid der Formel ArCOHal (III), wobei Ar wie für die Verbindung der Formel (I) definiert ist und Hal ein Halogenatom darstellt, behandelt wird;
b) die so erhaltene Verbindung der Formel: 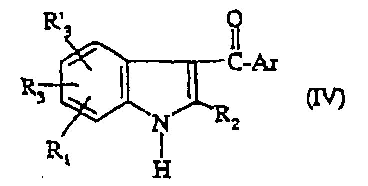 mit einem Halogenid der Formel Hal-A-Y (V), wobei -A- und Y wie für eine Verbindung der Formel (I) nach Anspruch 1 definiert sind und Hal ein Halogenatom darstellt, in Gegenwart einer Base behandelt wird.

11. Verfahren nach Anspruch 10, **dadurch gekennzeichnet, dass** Schritt b) auf folgende Weise modifiziert wird:
b1) die in Schritt a) erhaltene Verbindung der Formel: 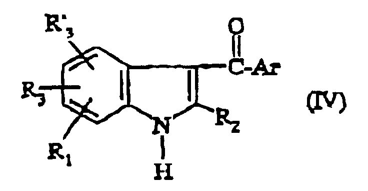 wird mit einer Verbindung der Formel Z-A-Cl (VI) behandelt, wobei Z entweder eine Hydroxylgruppe oder ein Halogenatom darstellt und -A- wie für (I) bei Anspruch 1 definiert ist;
b2) die so erhaltene Verbindung der Formel 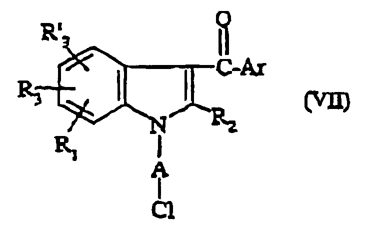 wird gegebenenfalls mit Natriumiodid behandelt;
b3) die so in Schritt b1) der Formel (VII) oder in Schritt b2) der Formel: 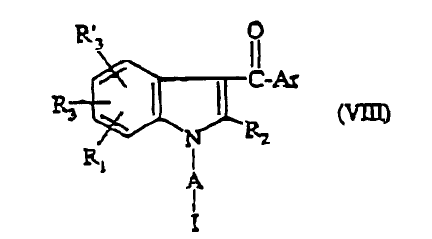 erhaltene Verbindung wird mit einem Anion Y^{⊖}, wobei Y wie für eine Verbindung der Formel (I) nach Anspruch 1 definiert ist, behandelt;

12. Verfahren nach Anspruch 10 zur Herstellung einer Verbindung der Formel 1, wobei Y eine Gruppe SOR₄ oder eine Gruppe SO₂R₄ darstellt, ausgehend von einer Verbindung der Formel (I), wobei Y eine Gruppe SR₄ darstellt, **dadurch gekennzeichnet, dass** der folgende zusätzliche Schritt durchgeführt wird:
c1) die erhaltene Verbindung der Formel: 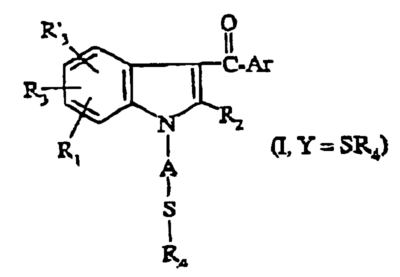 wird mit einem Oxidationsmittel behandelt.

13. Verfahren nach Anspruch 10 zur Herstellung einer Verbindung der Formel (I), wobei Y eine Gruppe N(R₇)SO₂R₄ darstellt, wobei R₇ von H verschieden ist, ausgehend von einer Verbindung der Formel (I), wobei Y eine Gruppe NHSO₂R₄ darstellt, **dadurch gekennzeichnet dass** der folgende zusätzliche Schritt durchgeführt wird:
c2) die erhaltene Verbindung der Formel: 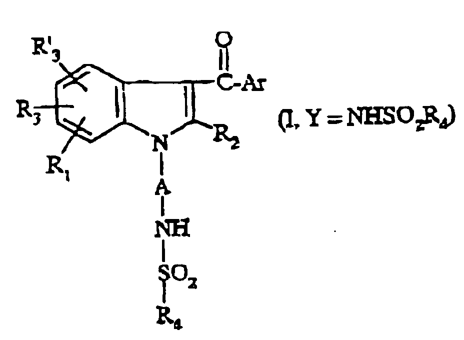 wird mit einem Alkylierungsmittel in Gegenwart einer Base behandelt.

14. Verfahren nach Anspruch 10 zur Herstellung einer Verbindung der Formel (I), wobei Y eine Gruppe SO₂NR₅R₆ darstellt, ausgehend von einer Verbindung der Formel (I),
wobei Y eine Gruppe SO₂NHR₅ darstellt, **dadurch gekennzeichnet, dass** der folgende zusätzliche Schritt durchgeführt wird:
c3) die erhaltene Verbindung der Formel: 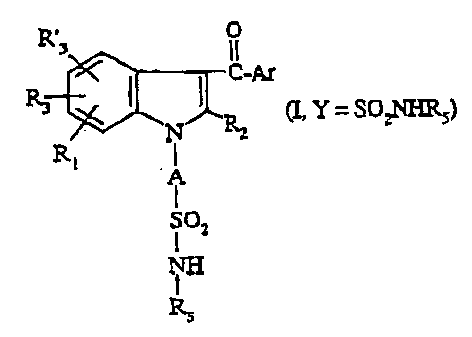 wird mit einem Alkylierungsmittel in Gegenwart einer Base behandelt.

15. Verfahren nach Anspruch 10 zur Herstellung einer Verbindung der Formel (I), wobei Y eine Gruppe NR₇SO₂R₄ oder eine Gruppe NR₇SO₂NR₅R₆ darstellt, **dadurch gekennzeichnet, dass**:
b4) die Verbindung der Formel: 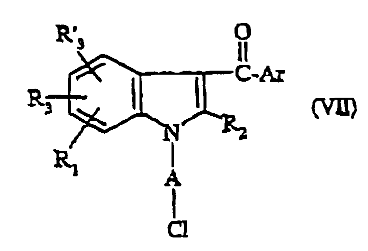 in eine Verbindung der Formel 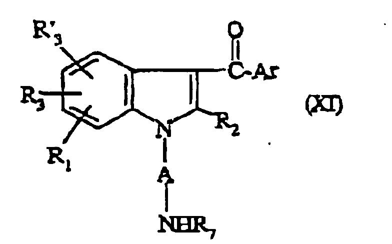 übergeführt wird, wobei R₇ wie für (I) definiert ist;
c4) eine Behandlung durch ein Halogenid der Formel HalSO₂R₄ bzw. HalSO₂NR₅R₆,
wobei R₄, R₅ und R₆ die nach Anspruch 1 für eine Verbindung der Formel 1 gegebene Bedeutung besitzen, erfolgt.

16. Verfahren zur Herstellung einer Verbindung der Formel (I) nach Anspruch 1, ihrer Salze und/oder Solvate, **dadurch gekennzeichnet, dass**:
i) ein Indol der Formel: 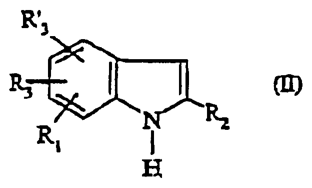 wobei R₁, R₂, R₃ und R'₃ wie für die Verbindung der Formel (I) definiert sind, mit einem Halogenid der Formel Hal-A-Y (V), wobei -A- und Y wie für eine Verbindung der Formel (I) nach Anspruch 1 definiert sind und Hal ein Halogenatom, vorzugsweise Brom, darstellt, in Gegenwart einer Base behandelt wird;
ii) die so erhaltene Verbindung der Formel 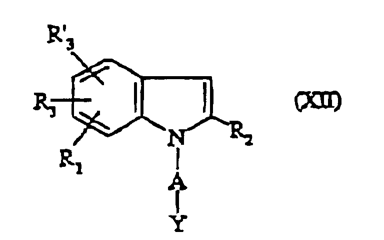 mit einem Säurehalogenid der Formel ArCOHal (III), wobei Ar wie für die Verbindung der Formel (I) nach Anspruch 1 definiert ist und Hal ein Halogenatom ist, behandelt wird.

17. Verfahren nach Anspruch 16, **dadurch gekennzeichnet, dass**
i1) ein Indol der Formel 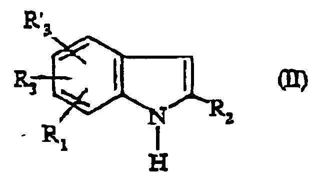 wobei R₁, R₂, R₃ und R'₃ wie für die Verbindung der Formel (I) definiert sind, mit einer Verbindung der Formel Z-A-Cl (VI), wobei -A- wie für die Verbindung der Formel (I) nach Anspruch 1 definiert ist und Z eine Hydroxylgruppe oder ein Halogenatom darstellt, behandelt wird;
i2) die so erhaltene Verbindung der Formel: 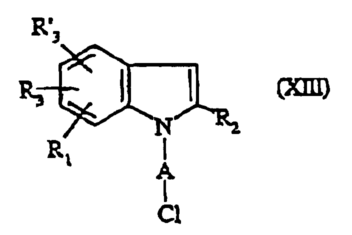 gegebenenfalls mit Natriumiodid behandelt wird;
i3) die so in Schritt i1) oder in Schritt i2) erhaltene Verbindung der Formel 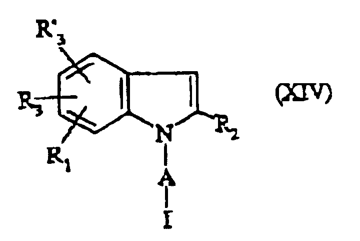 mit einem Anion der Formel Y behandelt wird, wobei Y wie für eine Verbindung der Formel (I) nach Anspruch 1 definiert ist;
ii) die so erhaltene Verbindung der Formel: 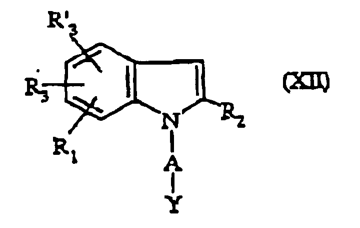 mit einem Säurehalogenid der Formel ArCOHal (III) behandelt wird, wobei Ar wie für die Verbindung der Formel (I) nach Anspruch 1 definiert ist und Hal ein Halogenatom ist.

18. Verbindung der Formel: 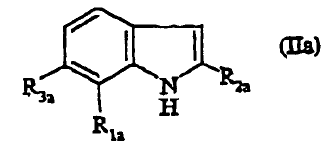 wobei
- R₁ₐ ein Chloratom oder Bromatom oder Methyl darstellt;
- R₂ₐ (C₁-C₄)-Alkyl darstellt;
- R₃ₐ Wasserstoff, ein Chlor- oder Bromatom oder Methyl darstellt;

19. Verbindung der Formel 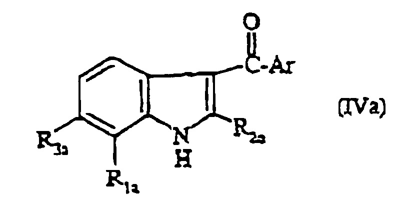 wobei
- R₁ₐ ein Chlor- oder Bromatom oder Methyl darstellt.
- R₂ₐ (C₁-C₄)-Alkyl darstellt;
- R₃ₐ Wasserstoff, ein Chlor- oder Bromatom oder Methyl darstellt;
- Ar wie für die Verbindungen der Formel (I) definiert ist.

20. Verbindung der Formel 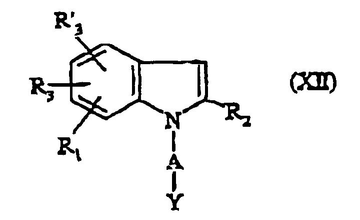 wobei R₁, R₂, R₃, R'₃ und Y wie für (I) nach Anspruch 1 definiert sind.

21. Pharmazeutische Zubereitung, die als Wirkstoff eine Verbindung nach einem der Ansprüche 1-9 enthält.

22. Pharmazeutische Zubereitung nach Anspruch 21, die 0,1-1000 mg Wirkstoff in Form einer Dosierungseinheit, in der der Wirkstoff mit mindestens einem pharmazeutischen Exzipiens gemischt ist, enthält.

23. Medikament, **dadurch gekennzeichnet, dass** es aus einer Verbindung nach einem der Ansprüche 1-9 besteht.

24. Verwendung einer Verbindung nach einem der Ansprüche 1-9 zur Herstellung von Medikamenten, die dazu bestimmt sind, jede Pathologie zu bekämpfen, an der die Cannabinoidrezeptoren CB₂ beteiligt sind.

## Claims

1. Compound of formula: 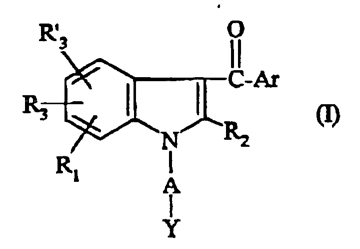 in which:
- Ar represents:
a) a phenyl mono-, di- or trisubstituted by one or more groups chosen from : a halogen atom, a (C₁-C₄)alkyl, a trifluoromethyl, an amino, a nitro, a hydroxyl, a (C₁-C₄)alkoxy, a (C₁-C₄)alkylsulphanyl or a (C₁-C₄)alkylsulphonyl;
b) a naphthyl which is unsubstituted or substituted once or twice by a halogen atom, a (C₁-C₄)alkyl or a trifluoromethyl ;
- A represents a C₂-C₆ alkylene radical ;
- Y represents a group chosen from SR₄, SOR₄, SO₂R₄, SO₂NR₅R₆, N(R₇)SO₂R₄, OR₄ or NR₇SO₂NR₅R₆ ;
- R₁, R₃ and R'₃ represent, each independently of one another, hydrogen, a hydroxyl, a halogen atom, a (C₁-C₄)alkyl, a trifluoromethyl or a (C₁-C₄)alkoxy ;
- R₂ represents hydrogen or a (C₁-C₄)alkyl ;
- R₄ represents a ⁻(C₁-C₄)alkyl or a trifluoromethyl ;
- R₅ and R₆ each independently represent hydrogen or a (C₁-C₄)alkyl ;
- R₇ represents hydrogen or a (C₁-C₄)alkyl ;
and its optional salts and/or its solvates.

2. Compound according to Claim 1 of formula: 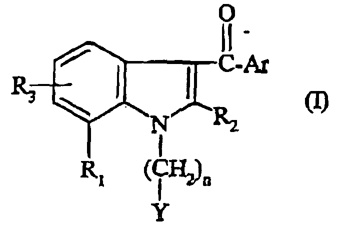 in which:
- Ar represents:
a) a phenyl mono-, di- or trisubstituted by one or more groups chosen from: a halogen atom, a (C₁-C₄)alkyl, a trifluoromethyl, an amino, a nitro, a (C₁-C₄)alkoxy, a (C₁-C₄)alkylsulphanyl or a (C₁-C₄)alkylsulphonyl;
b) a naphthyl which is unsubstituted or substituted once or twice by a halogen atom, a (C₁-C₄)alkyl or a trifluoromethyl ;
- n represents 2, 3 or 4 ;
- Y represents a group chosen from SR₄, SOR₄, SO₂R₄, SO₂NR₅R₆, N(R₇)SO₂R₄ or OR₄ ;
- R₁ represents a halogen atom, a (C₁-C₄)alkyl, a trifluoromethyl or a (C₁-C₄)alkoxy;
- R₂ represents hydrogen or a (C₁-C₄)alkyl ;
- R₃ represents hydrogen, a (C₁-C₄)alkyl or a halogen ;
- R₄ represents a (C₁-C₄)alkyl;
- R₅ and R₆ each independently represent hydrogen or a (C₁-C₄)alkyl ;
- R₇ represents hydrogen or a (C₁-C₄)alkyl ;
and its optional salts and/or its solvates.

3. Compound according to Claim I of formula (I) in which R₁ is in the 7 position of the indole nucleus and represents a methyl or a chlorine or bromine atom.

4. Compound according to Claim 1 of formula (I) in which R₂ represents a (C₁-C₄)alkyl.

5. Compound according to Claim 1 of formula (I) in which R₃ is hydrogen or R₃ is in the 6 position of the indole nucleus and represents either a chlorine atom or a methyl.

6. Compound according to Claim 1 of formula (I) in which R'₃ is hydrogen.

7. Compound according to Claim 1 of formula (I) in which Ar represents a phenyl mono- or disubstituted by a halogen atom, a methyl, a trifluoromethyl, a methoxy, a methylsulphanyl or a methylsulphonyl.

8. Compound according to Claim 1 of formula (I) in which Y represents SO₂R₄ or NHSO₂R₄.

9. Compound according to Claim 1 of formula (I) in which:
- Ar represents a phenyl mono- or disubstituted by a halogen atom, a methyl, a trifluoromethyl, a methoxy, a methylsulphanyl or a methylsulphonyl;
- A represents a (CH₂)ₙ group;
- n represents 2, 3 or 4 ;
- Y represents SO₂R₄ or NHSO₂R₄ ;
- R₁ represents a methyl or a chlorine or bromine atom in the 7 position of the indole nucleus;
- R₂ represents a methyl ;
- R₃ is hydrogen or R₃ represents either a chlorine atom or a methyl in the 6 position of the indole nucleus; .
- R'₃ is hydrogen;
- R₄ represents a methyl or an ethyl;
and its optional salts and/or its solvates.

10. Process for the preparation of a compound of formula (I) according to Claim 1, of its optional salts and/or its solvates, **characterized in that**:
a) an indole of formula: 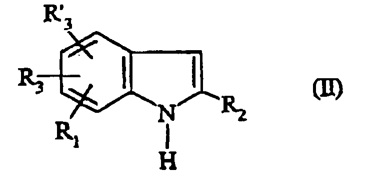 in which R₁, R₂, R₃ and R'₃ are as defined in Claim 1 for a compound of formula (I), is treated with a methylmagnesium halide and with an acid halide of formula ArCOHal (III), in which Ar is as defined for the compound of formula (I) and Hal represents a halogen atom;
b) the compound thus obtained, of formula: 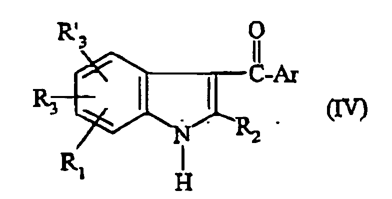 is treated with a halide of formula Hal-A-Y (V), in which -A- and Y are as defined for a compound of formula (I) in Claim 1 and Hal represents a halogen atom, in the presence of a base.

11. Process according to Claim 10, **characterized in that** stage b) is modified in the following way:
b1) the compound obtained in stage a), of formula: 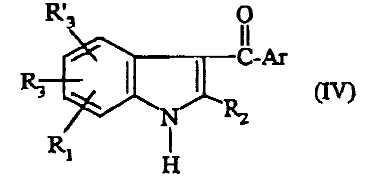 is treated with a compound of formula Z-A-CL (VI), in which Z represents either a hydroxyl group or a halogen atom and -A- is as defined for (I) in Claim 1;
b2) optionally, the compound thus obtained, of formula: 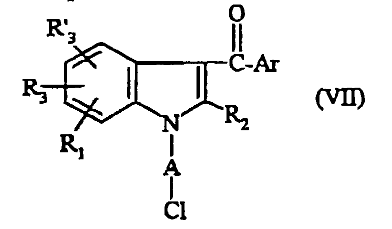 is treated with sodium iodide;
b3) the compound thus obtained in stage b1), of formula (VII), or in stage b2), of formula: 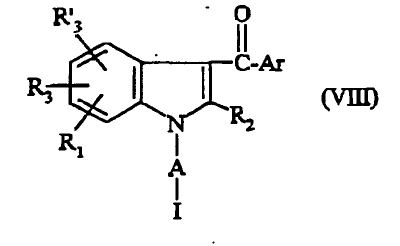 is treated with a Y^{⊖} anion, Y being as defined for a compound of formula (I) in Claim 1.

12. Process according to Claim 10 for preparing a compound of formula (I) in which Y represents an SOR₄ group or an SO₂R₄ group, from a compound of formula (I) in which Y represents an SR₄ group, **characterized in that** the following additional stage is carried out:
c1) the compound obtained, of formula: 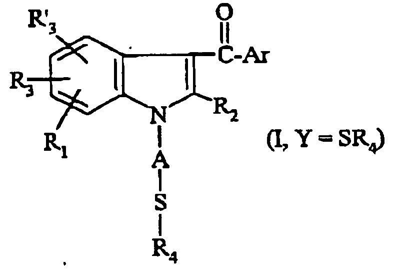 is treated with an oxidizing agent.

13. Process according to Claim 10 for preparing a compound of formula (I) in which Y represents an N(R₇) SO₂R₄ group with R₇ other than H, from a compound of formula (I) in which Y represents an NHSO₂R₄ group, **characterized in that** the following additional stage is carried out:
c2) the compound obtained, of formula: 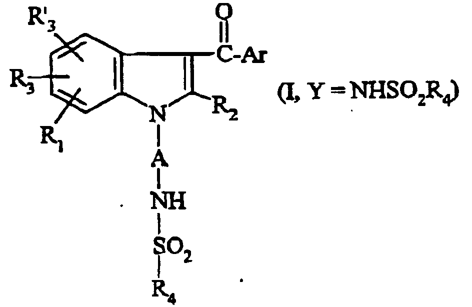 is treated with an alkylating agent in the presence of a base.

14. Process according to Claim 10 for preparing a compound of formula (I) in which Y represents an SO₂NR₅R₆ group from a compound of formula (I) in which Y represents an SO₂NHR₅ group, **characterized in that** the following additional stage is carried out:
c3) the compound obtained, of formula: 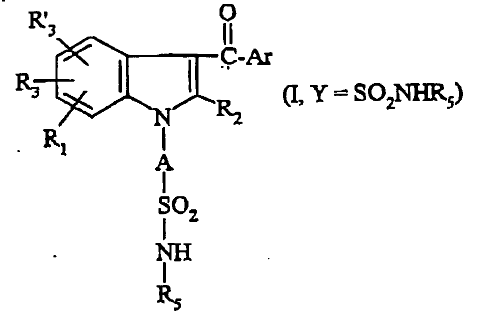 is treated with an alkylating agent in the presence of a base.

15. Process according to Claim 10 for preparing a compound of formula (I) in which Y represents an NR₇SO₂R₄ group or an NR₇SO₂NR₅R₆ group, **characterized in that**:
b4) the compound of formula: 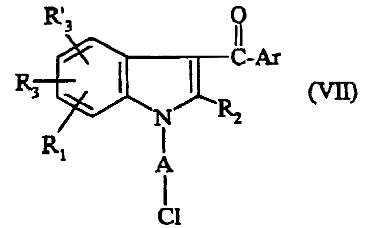 is converted into a compound of formula: 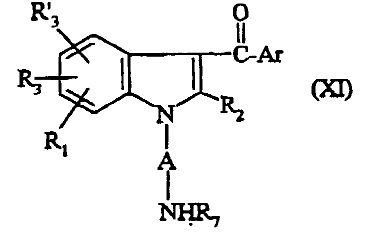 in which R₇ is as defined for (I);
c4) treatment is carried out with a halide of formula HalSO₂R₄ or respectively HalSO₂NR₅R₆ in which R₄, R₅ and R₆ have the meanings given in Claim 1 for a compound of formula (I).

16. Process for the preparation of a compound of formula (I) according to Claim 1, of its salts and/or solvates, **characterized in that**:
i) an indole of formula: 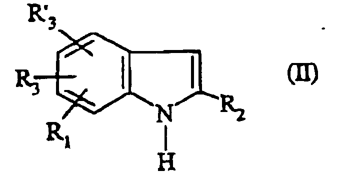 in which R₁, R₂, R₃ and R'₃ are as defined for the compound of formula (I), is treated with a halide of formula Hal-A-Y (V) in which -A- and Y are as defined for a compound of formula (I) in Claim 1 and Hal represents a halogen atom, preferably bromine, in the presence of a base;
ii) the compound thus obtained, of formula: 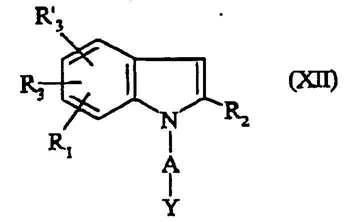 is treated with an acid halide of formula ArCOHal (III) in which Ar is as defined for the compound of formula (I) in Claim 1 and Hal is a halogen atom.

17. Process according to Claim 16, **characterized in that**:
i1) an indole of formula: 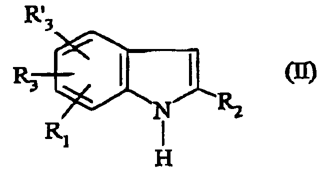 in which R₁, R₂, R₃ and R'₃ are as defined for the compound of formula (I), is treated with a compound of formula Z-A-Cl (VI) in which -A- is as defined for the compound of formula (I) in Claim 1 and Z represents a hydroxyl group or a halogen atom,
i2) optionally, the compound thus obtained, of formula: 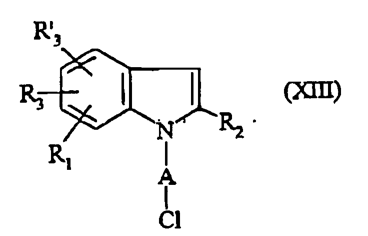 is treated with sodium iodide;
i3) the compound thus obained in stage i1) or in stage i2), of formula: 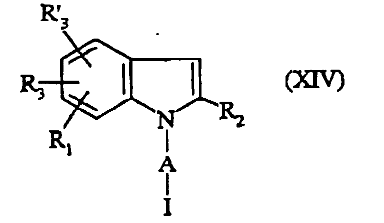 is treated with an anion of formula Y⁻, Y being as defined for a compound of formula (I) in Claim 1;
ii) the compound thus obtained, of formula: 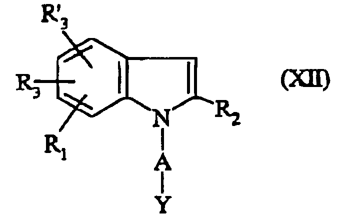 is treated with an acid halide of formula ArCOHal (III) in which Ar is as defined for the compound of formula (I) in Claim 1 and Hal is a halogen atom.

18. Compound of formula: 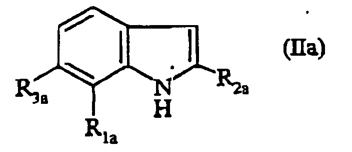 in which:
- R₁ₐ represents a chlorine or bromine atom or a methyl;
- R₂ₐ represents a (C₁-C₄)alkyl;
- R₃ₐ represents hydrogen, a chlorine or bromine atom, or a methyl.

19. Compound of formula: 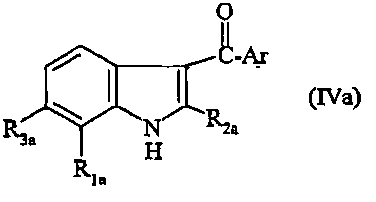 in which:
- R₁ₐ represents a chlorine or bromine atom or a methyl;
- R₂ₐ represents a (C₁-C₄)alkyl;
- R₃ₐ represents hydrogen, a chlorine or bromine atom, or a methyl;
- Ar is as defined for the compounds of formula (I).

20. Compound of formula: 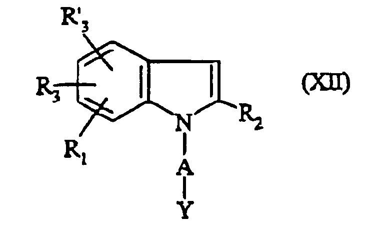 in which R₁, R₂, R₃, R'₃ and Y are as defined for (I) in Claim 1.

21. Pharmaceutical composition comprising, as active principle, a compound according to any one of Claims 1 to 9.

22. Pharmaceutical composition according to Claim 21 comprising from 0.1 to 1 000 mg of active principle in the form of a dosage unit in which the active principle is mixed with at least one pharmaceutical excipient.

23. Medicament, **characterized in that** it is composed of a compound according to any one of Claims 1 to 9.

24. Use of a compound according to any one of Claims 1 to 9 in the preparation of medicaments intended to combat any pathology in which CB₂ cannabinoid receptors are implicated.
